(19) Europäisches Patentamt
European Patent Office
Office européen des brevets

(11) EP 1 410 782 B1

(12) EUROPEAN PATENT SPECIFICATION

(45) Date of publication and mention of the grant of the patent:
**30.07.2008 Bulletin 2008/31**

(21) Application number: **02738774.5**

(22) Date of filing: **20.06.2002**

(51) Int Cl.:
***A61K 8/87*** *(2006.01)* ***A61Q 1/00*** *(2006.01)*
***A61Q 3/00*** *(2006.01)* ***A61Q 5/00*** *(2006.01)*
***A61Q 19/00*** *(2006.01)*

(86) International application number:
**PCT/JP2002/006169**

(87) International publication number:
**WO 2003/002073 (09.01.2003 Gazette 2003/02)**

# (54) COSMETIC COMPOSITION COMPRISING AN AMPHIPHILIC URETHANE RESIN

KOSMETISCHE ZUSAMMENSETZUNG ENTHALTEND EIN AMPHIPHILES URETHANHARZ

COMPOSITION COSMETIQUE COMPRENANT UNE RESINE URETHANE AMPHIPHILE

(84) Designated Contracting States:
**DE FR GB IT**

(30) Priority: **27.06.2001 JP 2001193963**

(43) Date of publication of application:
**21.04.2004 Bulletin 2004/17**

(73) Proprietor: **SHISEIDO COMPANY, LTD.**
**Tokyo 104-8010 (JP)**

(72) Inventors:
- **OMURA, Takayuki,**
**Shiseido Res.Ctr. (Shin-Yokohama)**
**Yokohama-shi,**
**Kanagawa 224-8558 (JP)**
- **SHIDA, Tomotaka,**
**Shiseido Res.Ctr. (Shin-Yokohama)**
**Yokohama-shi,**
**Kanagawa 224-8558 (JP)**
- **NANBA, Tomiyuki,**
**Shiseido Res.Ctr. (Shin-Yokohama)**
**Yokohama-shi,**
**Kanagawa 224-8558 (JP)**

(74) Representative: **Merkle, Gebhard et al**
**TER MEER STEINMEISTER & PARTNER GbR,**
**Patentanwälte,**
**Mauerkircherstrasse 45**
**81679 München (DE)**

(56) References cited:
**WO-A-01/10393** **WO-A-01/10394**
**WO-A-01/10397** **WO-A-02/09658**
**WO-A-99/39688** **WO-A1-01/10397**
**WO-A2-01/10393** **WO-A2-01/10394**
**JP-A- 2000 191 476** **JP-A- 2002 020 221**
**JP-A- 2002 020 451**

- **DATABASE WPI Section Ch, Week 200233 Derwent Publications Ltd., London, GB; Class A25, AN 2002-287397 XP002344079 & JP 2002 020221 A (SHISEIDO CO LTD) 23 January 2002 (2002-01-23)**

EP 1 410 782 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention relates to a cosmetic formulation comprising an amphiphilic urethane resin carrying a polysiloxane compound.

### BACKGROUND OF THE INVENTION

There are a variety of cosmetic formulations in which coating characteristics are utilized, including hair cosmetic formulations, nail formulations, facial pack formulations, eye formulations and the like. For example, a hair formulation contains, as an essential component, a hair-styling component which has been selected from anionic, nonionic or amphiphilic acrylic polymers, vinyl pyrrolidone-based polymers, cationic vinyl pyrrolidone-based or cellulose-based polymers and the like. A nail formulation contains nitrocellulose, an acrylic polymer and the like as a film-forming agent or film-forming aid and a facial pack formulation contains a polyvinyl alcohol, polyvinyl acetate and the like as a film forming agent, while an eye formulation such as a mascara and eyeliner contains an acrylic polymer, polyvinyl acetate and the like as a film-forming agent.

However, any of the materials described above is not a film-forming agent which is satisfactory for providing a product which meets a consumer's demand.

JP-A-11-228363 discloses a cosmetic resin composition containing an amphiphilic urethane resin. JP-A-2000-191476 discloses an amphiphilic urethane resin obtained by introducing a polysiloxane chain into the backbone of the amphiphilic urethane resin using a polysiloxane compound having an active hydrogen-containing functional group on the both or either one of the terminals of the siloxane chain. JP-A-2001-48735 discloses a cosmetic formulation into which an amphiphilic urethane resin and a silicone polymer are incorporated each as a formulation component. However, any of these cosmetic formulations and resin-containing formulations is not satisfactory in terms of the handling performance and the stability.

Under such circumstances, the present invention is intended to develop a coating agent consisting of a novel amphiphilic urethane resin obtained by improving a conventional urethane resin and to apply such a formulation to a cosmetic product.

### SUMMARY OF THE INVENTION

Now we discovered that the problems discussed above can be solved by developing a novel coating agent in which a polysiloxane compound is carried on an amphiphilic urethane resin, whereby establishing the invention.

Thus, the invention is a cosmetic formulation comprising an amphiphilic urethane resin carrying a polysiloxane compound according to claim 1.

A novel amphiphilic urethane resin employed in the invention is characterized structurally by a polysiloxane compound carried thereon.

The phrase "carrying" a polysiloxane here means "restricting" a polysiloxane compound by an amphiphilic urethane resin backbone, or "tangling" a polysiloxane compound with an amphiphilic urethane resin backbone, rather than binding a polysiloxane compound chemically with an amphiphilic urethane resin backbone. Accordingly, the term "carried" used herein means that a polysiloxane compound is "restricted" by an amphiphilic urethane resin backbone or that a polysiloxane compound is "tangled" with an amphiphilic urethane resin backbone. The carrying of a polysiloxane compound on an amphiphilic urethane resin can be identified for example by IR spectroscopy.

The amphiphilic urethane resin as a constituent of the amphiphilic urethane resin carrying a polysiloxane compound employed in a cosmetic formulation of the invention is an amphiphilic urethane resin formed by reacting at least compounds (A) to (D):

(A) a polyol compound;
(B) a polyisocyanate compound;
(C) a compound having at least one group selected from a hydroxyl group, primary amino group and secondary amino group and also having a carboxyl group; and,
(D) a compound having at least one group selected from a hydroxyl group, primary amino group and secondary amino group and also having a tertiary amino group.

To exist means here that a polysiloxane compound just exists in the reaction system. The term "exist" in conjunction with the production of an inventive amphiphilic urethane resin carrying a polysiloxane compound is employed here to have the meaning described above.

The amphiphilic urethane resin carrying a polysiloxane compound described above which is employed preferably can be obtained using at least compounds (A) to (D) and (S):

(A) a polyol compound;
(B) a polyisocyanate compound;
(C) a compound having at least one group selected from a hydroxyl group, primary amino group and secondary amino group and also having a carboxyl group;
(D) a compound having at least one group selected from a hydroxyl group, primary amino group and secondary amino group and also having a tertiary amino group; and,
(S) a polysiloxane compound as defined in claim 1;

by a method comprising a first step for producing an isocyanate group-containing prepolymer by reacting the compounds (A), (B) and (C) under an isocyanate group-excess condition and a second step for reacting said isocyanate group-containing prepolymer with the compound (D),
wherein the compound (S) is allowed to exist in at least one of the first step and second step.

The amphiphilic urethane resin carrying a polysiloxane compound described above which is employed preferably can be obtained using at least compounds (A) to (D) and (S):

(A) a polyol compound;
(B) a polyisocyanate compound;
(C) a compound having at least one group selected from a hydroxyl group, primary amino group and secondary amino group and also having a carboxyl group;
(D) a compound having at least one group selected from a hydroxyl group, primary amino group and secondary amino group and also having a tertiary amino group; and,
(S) a polysiloxane compound as defined in claim 1;

by a method comprising a first step for producing an isocyanate group-containing prepolymer by reacting the compounds (A), (B) and (D) under an isocyanate group-excess condition and a second step for reacting said isocyanate group-containing prepolymer with the compound (C),
wherein the compound (S) is allowed to exist in at least one of the first step and second step.

In the production of an inventive amphiphilic urethane resin carrying a polysiloxane compound, a polysiloxane compound is allowed to exist in either of the first step and the second step.

In producing an inventive amphiphilic urethane resin carrying a polysiloxane compound using at least the compounds (A) to (D) and (S) described above, it is preferable that after the second step a step for mixing the reaction product from the second step with water to perform a chain elongation reaction is further provided.

It is further preferred in producing an inventive amphiphilic urethane resin carrying a polysiloxane compound using at least the compounds (A) to (D) and (S) described above that after the second step a step for mixing the reaction product from the second step with basic water to perform a chain elongation reaction or a step for adding a basic compound to the reaction product from the second step followed by mixing with water to perform a chain elongation reaction is further provided.

The amphiphilic urethane resin carrying a polysiloxane compound employed in the invention may be an amphiphilic urethane resin formed by reacting in the presence of a polysiloxane compound at least compounds (A) to (E):

(A) a polyol compound;
(B) a polyisocyanate compound;
(C) a compound having at least one group selected from a hydroxyl group, primary amino group and secondary amino group and also having a carboxyl group;
(D) a compound having at least one group selected from a hydroxyl group, primary amino group and secondary amino group and also having a tertiary amino group; and,
(E) a compound having at least one group selected from a hydroxyl group, primary amino group and secondary amino group and also having a structural unit represented by Formula (1):

$$-(C_2H_4O)_p(C_3H_6O)_q- \qquad (1)$$

wherein p is an integer of 1 to 500 and q is an integer of 0 to 400.

The amphiphilic urethane resin carrying a polysiloxane compound described above which is employed preferably can be obtained using at least compounds (A) to (E) and (S):

(A) a polyol compound;
(B) a polyisocyanate compound;
(C) a compound having at least one group selected from a hydroxyl group, primary amino group and secondary amino group and also having a carboxyl group;
(D) a compound having at least one group selected from a hydroxyl group, primary amino group and secondary amino group and also having a tertiary amino group;
(E) a compound having at least one group selected from a hydroxyl group, primary amino group and secondary amino group and also having a structural unit represented by Formula (1):

$$-(C_2H_4O)_p(C_3H_6O)_q- \qquad (1)$$

wherein p is an integer of 1 to 500 and q is an integer of 0 to 400; and,
(S) a polysiloxane compound as defined in claim 1;

by a method comprising a first step for producing an isocyanate group-containing prepolymer by reacting the compounds (A), (B), (C) and (E) under an isocyanate group-excess condition and a second step for reacting said isocyanate group-containing prepolymer with the compound (D),
wherein the compound (S) is allowed to exist in at least one of the first step and second step.

Furthermore, the amphiphilic urethane resin carrying a polysiloxane compound described above which is employed preferably can be obtained using at least compounds (A) to (E) and (S):

(A) a polyol compound;
(B) a polyisocyanate compound;
(C) a compound having at least one group selected from a hydroxyl group, primary amino group and secondary amino group and also having a carboxyl group;
(D) a compound having at least one group selected from a hydroxyl group, primary amino group and secondary amino group and also having a tertiary amino group;
(E) a compound having at least one group selected from a hydroxyl group, primary amino group and secondary amino group and also having a structural unit represented by Formula (1):

$$-(C_2H_4O)_p(C_3H_6O)_q- \qquad (1)$$

wherein p is an integer of 1 to 500 and q is an integer of 0 to 400; and,
(S) a polysiloxane compound as defined in claim 1;

by a method comprising a first step for producing an isocyanate group-containing prepolymer by reacting the compounds (A), (B), (D) and (E) under an isocyanate group-excess condition and a second step for reacting said isocyanate group-containing prepolymer with the compound (C),
wherein the compound (S) is allowed to exist in at least one of the first step and second step.

In producing an inventive amphiphilic urethane resin carrying a polysiloxane compound using at least the compounds (A) to (E) and (S) described above, it is preferable that after the second step a step for mixing the reaction product from the second step with water is performed to effect a chain elongation reaction.

Moreover, in producing an inventive amphiphilic urethane resin carrying a polysiloxane compound using at least the compounds (A) to (E) and (S) described above, it is preferable that after the second step a step for mixing the reaction product from the second step with basic water is performed to effect a chain elongation reaction or a step for adding a basic compound to the reaction product from the second step followed by mixing with water is performed to effect a chain elongation reaction.

In producing an inventive amphiphilic urethane resin carrying a polysiloxane compound, a polysiloxane compound is allowed to exist in at least one of the first step and the second step. The polysiloxane compound employed here may exist at any time during the first step and the second step, and the polysiloxane compound is not necessary to exist at an early stage of the reaction. It is sufficient that the polysiloxane compound exists not later than the mixing of the reaction product from the second step with water. Accordingly, in the invention, "the first step" covers the duration from the initiation of the first step through the initiation of the second step, while "the second step" covers the duration from the initiation of the second step through the initiation of the subsequent step (more typically, the step for mixing the reaction product from the second step with water as described below).

A polysiloxane compound employed in the production of an inventive amphiphilic urethane resin carrying a polysiloxane compound is preferably a polysiloxane compound which does not have, on both or either one of the terminals of the siloxane, at least one group selected from a hydroxyl group, primary amino group and secondary amino group.

A polysiloxane compound employed in the production of an inventive amphiphilic urethane resin carrying a polysiloxane compound is one or more selected from a dimethylpolysiloxane, polyether-modified silicone, cyclic silicone, phenyl-modified silicone, alkyl-modified silicone and alkoxy-modified silicone.

An inventive amphiphilic urethane resin carrying a polysiloxane compound is incorporated preferable as an aqueous liquid of said amphiphilic urethane.

An inventive amphiphilic urethane resin is preferably an amphiphilic urethane resin having a carboxyl group and a tertiary amino group in one molecule.

A cosmetic formulation to which the invention is applied preferably is a hair formulation and a dermal external formulation.

DESCRIPTION OF THE PREFERRED EMBODIMENTS

The embodiments of the invention are described below.

A cosmetic formulation of the invention contains an amphiphilic urethane resin carrying a polysiloxane compound. The amphiphilic urethane resin described above which is employed preferably in the invention is an amphiphilic urethane resin having a carboxyl group and a tertiary amino group in one molecule.

An amphiphilic urethane resin as a constituent of an inventive amphiphilic urethane resin carrying a polysiloxane compound having a carboxyl group and a tertiary amino group in one molecule (hereinafter sometimes referred to as a polysiloxane-carrying amphiphilic urethane resin) is an amphiphilic urethane resin formed by reacting at least compounds (A) to (D):

(A) a polyol compound;
(B) a polyisocyanate compound;
(C) a compound having at least one group selected from a hydroxyl group, primary amino group and secondary amino group and also having a carboxyl group; and,
(D) a compound having at least one group selected from a hydroxyl group, primary amino group and secondary amino group and also having a tertiary amino group.

A polyol compound employed in the invention (hereinafter referred to as a compound (A)) is not limited particularly provided that it is a polyol compound employed ordinarily in producing an urethane resin. The compound (A) may for example be a polyester polyol, polyether polyol, low molecular weight polyol, polycarbonate polyol, polybutadiene polyol, polyisoprene polyol, polyolefin polyol, polyacrylate-based polyol and the like, any of which can be employed alone or in combination. Among those listed above, a polyester polyol, polyether polyol and low molecular weight polyol are employed preferably.

Such a polyester polyol may for example be a polyester polyol obtained by condensation polymerization of at least one dicarboxylic acid such as succinic acid, glutaric acid, adipic acid, sebacic acid, azelaic acid, maleic acid, fumaric acid, phthalic acid and terephthalic acid with at least one polyhydric acid such as ethylene glycol, propylene glycol, 1,4-butanediol, 1,3-butanediol, 1,6-hexanediol, neopentyl glycol, 1,8-octanediol, 1,10-decanediol, diethylene glycol, spiroglycol and trimethylol propane, as well as a polyester polyol obtained by ring-opening polymerization of a lacton.

A polyether polyol mentioned above may for example be a polyether polyol obtained by ring-opening addition polymerization of water and a polyhydric alcohol employed in the synthesis of a polyester polyol described above as well as a phenol such as bisphenol A and a hydride thereof, a primary amine or secondary amine, with a cyclic ether such as ethylene oxide, propylene oxide, oxethane and tetrahydrofuran. Those also exemplified include a polyoxypropylene polyol, polyoxytetramethylene polyol, as well as a polyether polyol obtained by ring-opening addition polymerization of bisphenol A with at least one of propylene oxide and ethylene oxide (when a copolymer results, it may be a block copolymer or random copolymer).

A low molecular weight polyol mentioned above may for example be 1,4-cyclohexanedimethanol, ethylene glycol, propylene glycol, isopropylene glycol, 1,4-butanediol, 1,3-butanediol, butylene glycol, 1,6-hexanediol, neopentyl glycol, 1,8-octanediol, 1,10-decanediol, diethylene glycol, dipropylene glycol, spiroglycol, trimethylol propane, glycerin, diglycerin, triglycerin and the like.

A compound (A) may be employed alone or in combination. A preferred compound (A) is 1,4-cyclohexane dimethanol.

In the invention, a compound (A), which overlaps with a compound (E) described below, is included in a compound (E) rather than in a compound (A). Also in the invention, a compound (A), which overlaps with a compound (C) described below, is included in a compound (C) rather than in a compound (A). Furthermore in the invention, a compound (A), which overlaps with a compound (D) described below, is included in a compound (D) rather than in a compound (A).

A polyisocyanate compound employed in the invention (hereinafter sometimes referred to as a compound (B))

is not limited particularly provided that it is a polyisocyanate compound employed ordinarily in producing an urethane resin. The compound (B) may for example be an organic diisocyanate compound such as an aliphatic diisocyanate compound, alicyclic diisocyanate compound and aromatic diisocyanate compound, which can be employed alone or in combination with each other.

An aliphatic diisocyanate mentioned above may for example be ethylene diisocyanate, 2,2,4-trimethylhexamethylene diisocyanate and 1,6-hexamethylene diisocyanate.

An alicyclic diisocyanate mentioned above may for example be hydrogenated 4,4'-diphenylmethane diisocyanate, 1,4-cyclohexane diisocyanate, methylcyclohexylene diisocyanate, isophorone diisocyanate and norbornane diisocyanate.

An aromatic diisocyanate mentioned above may for example be 4,4'-diphenylmethane diisocyanate, xylylene diisocyanate, toluene diisocyanate and naphthalene diisocyanate.

Among the compounds (B) listed above, 1,6-hexamethylene diisocyanate, isophorone diisocyanate and norbornane diisocyanate and the like are preferred because of excellent weatherability and less expense. Any of compounds (b) may be employed alone or in combination.

A compound having at least one group selected from a hydroxyl group, primary amino group and secondary amino group and also having a carboxyl group (hereinafter sometimes referred to as a compound (C)) employed in the invention is not limited particularly provided that it is a compound having at least one group selected from a hydroxyl group, primary amino group and secondary amino group and also having at least one carboxyl group and can give an intended amphiphilic urethane resin. The compound (C) may preferably be a carboxylic acid of 3 to 26, preferably 3 to 12 carbon atoms having a dialkylol group such as dimethylol, diethanol and dipropanol. Those exemplified typically include dimethylol propanoic acid (DMPA) and dimethylol butanoic acid. A carboxyl group-containing polycaprolactonediol can also be employed. Those listed above may be employed alone or in combination.

A compound having at least one group selected from a hydroxyl group, primary amino group and secondary amino group and also having a tertiary amino group (hereinafter sometimes referred to as a compound (D)) employed in the invention is not limited particularly provided that it is a compound having at least one group selected from a hydroxyl group, primary amino group and secondary amino group and also having at least one tertiary amino group and can give an intended amphiphilic urethane resin. The compound (D) may for example be an N-alkyldialkanolamine compound one having a dialkylol group similar to a compound (C) such as N-methyldiethanolamine, N-ethyldiethanolamine, N-butyldiethanolamine, N-lauryldiethanolamine and N-methyldipropanolamine. The number of carbon atoms in the alkyl group of an N-alkyl in an N-alkyldialkanolamine is preferably 1 to 24, especially 1 to 8. Those also exemplified are an N,N-dialkylalkanolamine compound such as N,N-dimethylethanolamine, N,N-diethylethanolamine and N,N-dibutylethanolamine as well as triethanolamine. Those listed above may be employed alone or in combination.

A polysiloxane compound employed in the invention (hereinafter sometimes referred to as a compound (S)) is at least one selected from dimethylpolysiloxane, polyether-modified silicone, cyclic silicone, phenyl-modified silicone, alkyl-modified silicone and alkoxy-modified silicone. These polysiloxane compounds may be employed alone or in combination.

A dimethylpolysiloxane may for example be a compound represented by Formula (2):

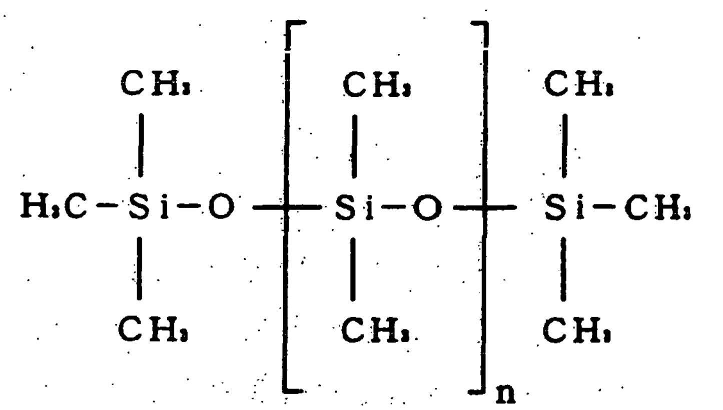

··· (2)

wherein n is an integer of 1 or more.

In the formula, n is preferably an integer of 1 to 100, more preferably 1 to 50, especially 3 to 30.

A dimethylpolysiloxane in the invention may be any commercially available one, such as SH 200 series (trade name) produced by Dow Corning Toray Silicone Co. Ltd., and as well as KF 96 series produced by Shin-Etsu Chemical Co. Ltd.

A polyether-modified silicone may for example be a compound represented by Formula (3):

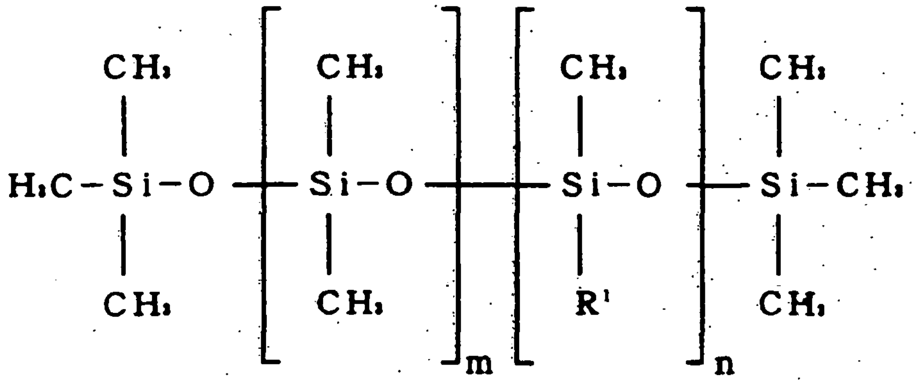

··· (3)

wherein m is an integer of 0 or more, n is an integer of 1 or more, and $R^1$ is a group represented by Formula (4):

$$-(CH_2)_a-(OC_2H_4)_b(OC_3H_6)_c-OR^2 \qquad (4)$$

wherein $R^2$ is a hydrogen atom or a hydrocarbon group having 1 to 10 carbon atoms, a is an integer of 1 to 10, b is an integer of 1 to 300 and c is an integer of 0 to 300.

In Formula (3), m is preferably an integer of 1 to 300, more preferably 1 to 100, particularly 1 to 50. n is preferably an integer of 1 to 300, more preferably 1 to 100, particularly 1 to 50. Also in Formula (4), a is preferably an integer of 1 to 5, particularly 2 to 4. b is preferably an integer of 2 to 50, more preferably 2 to 40, particularly 2 to 30. c is preferably an integer of 0 to 50, more preferably 0 to 40, particularly 0 to 30.

A compound (S) represented by Formula (3) which is preferred is a compound represented by Formula (3) wherein m is an integer of 1 to 300, n is an integer of 1 to 300, $R^1$ is a group represented by Formula (4), a is an integer of 1 to 5, b is an integer of 2 to 50 and c is an integer of 0 to 50.

A compound (S) represented by Formula (3) which is more preferred is a compound represented by Formula (3) wherein m is an integer of 1 to 100, n is an integer of 1 to 100, $R^1$ is a group represented by Formula (4), a is an integer of 2 to 4, b is an integer of 2 to 40 and c is an integer of 0 to 40.

A compound (S) represented by Formula (3) which is especially preferred is a compound represented by Formula (3) wherein m is an integer of 1 to 50, n is an integer of 1 to 50, $R^1$ is a group represented by Formula (4), a is an integer of 2 to 4, b is an integer of 2 to 30 and c is an integer of 0 to 30.

A polyether-modified silicone represented by Formula (3) may for example be SH3746, SH3771C, SH3772C, SH3773C, SH3775C, SH3748, SH3749, SH3771M, SH3772M, SH3773M and SH3775M (trade name) produced by Dow Corning Toray Silicone Co. Ltd., as well as KF351A, KF353A, KF945A, KF352A, KF615A, KF6011, KF6012, KF6013, KF6015, KF6016 and KF6017 (trade name) produced by Shin-Etsu Chemical Co. Ltd..

A phenyl-modified silicone may for example be a compound represented by Formula (5):

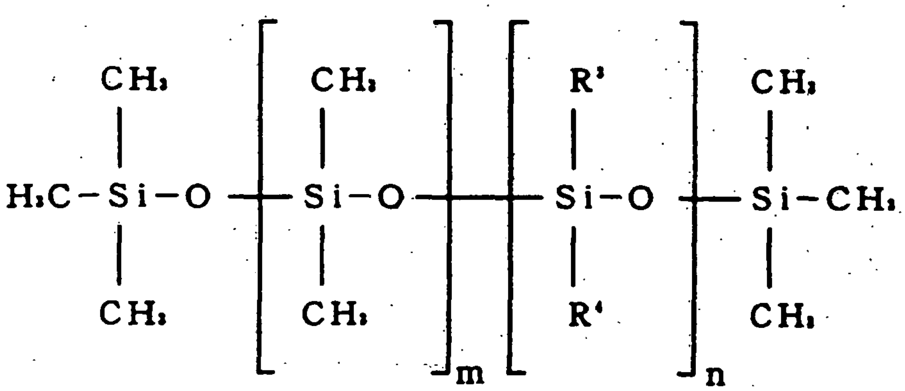

··· (5)

wherein each of $R^3$ and $R^4$, which may be same or different, is a hydrocarbon group having 1 to 12 carbon atoms (for example, a straight or branched saturated hydrocarbon group having 1 to 12 carbon atoms), $-OSi(CH_3)_3$ or phenyl group, provided that at least one of $R^3$ and $R^4$ is a phenyl group, m is an integer of 0 or more and n is an integer of 1 or more.

In Formula (5), m is preferably an integer of 1 to 300, more preferably 1 to 100, particularly 1 to 50. n is preferably an integer of 1 to 500, more preferably 1 to 100, particularly 1 to 50. A phenyl-modified silicone which is preferred especially is a methylphenylpolysiloxane represented by Formula (5) wherein $R^3$=$CH_3$ or $-OSi(CH_3)_3$, $R^4$=$C_6H_5$, m=0 and n=1 to 100.

A phenyl-modified silicone represented by Formula (5) may for example be SH556, SF557, SF558 and SH559 (trade name) produced by Dow Corning Toray Silicone Co. Ltd., and KF50-100cs, KF50-1000cs, KF53, KF54 and KF56 (trade name) produced by Shin-Etsu Chemical Co. Ltd.

An alkyl-modified silicone may for example be a compound represented by Formula (6):

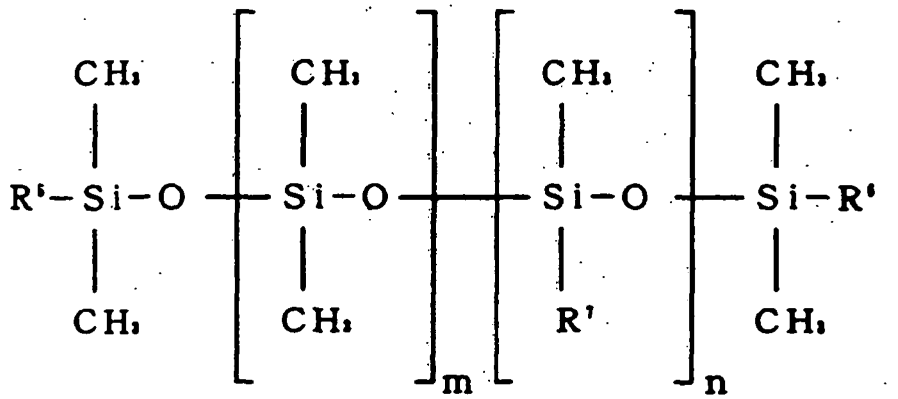

··· (6)

wherein each of $R^5$ to $R^7$, which may be same or different, is a hydrocarbon group having 1 to 50 carbon atoms, provided that at least one of $R^5$ to $R^7$ is a hydrocarbon group having 5 to 30 carbon atoms, m is an integer of 1 or more and n is an integer of 1 or more.

In Formula (6), each of $R^5$ to $R^7$ may for example be a straight or branched saturated hydrocarbon group having 1 to 50 carbon atoms. The number of carbon atoms in the hydrocarbon group is preferably 5 to 30, more preferably 5 to 20, particularly 1 to 20. m is preferably an integer of 10 to 300, more preferably 1 to 100, particularly 1 to 50. n is preferably an integer of 1 to 300, more preferably 1 to 100, particularly 1 to 50.

An alkyl-modified silicone represented by Formula (6) may for example be SF8416 (trade name) produced by Dow Corning Toray Silicone Co. Ltd., and KF-412, KF-413 and KF-414 (trade name) produced by Shin-Etsu Chemical Co. Ltd..

An alkoxy-modified silicone may for example be a compound represented by Formula (7):

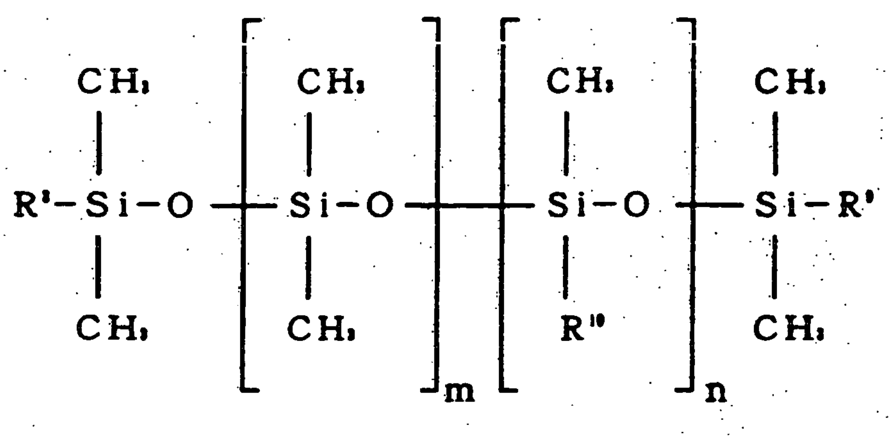

··· (7)

wherein each of $R^8$ to $R^{10}$, which may be same or different, is a hydrocarbon group having 1 to 12 carbon atoms or an

alkoxy group having 1 to 50 carbon atoms, provided that at least one of $R^8$ to $R^{10}$ is an alkoxy group having 1 to 50 carbon atoms, m is an integer of 0 or more, and n is an integer of 1 or more.

While each of $R^8$ to $R^{10}$ in Formula (7) is a hydrocarbon group having 1 to 12 carbon atoms or an alkoxy group having 1 to 50 carbon atoms, such a hydrocarbon group having 1 to 12 carbon atoms may for example be a straight or branched saturated hydrocarbon group and an alkoxy group having 1 to 50 carbon atoms may for example be a straight or branched alkoxy group. The number of carbon atoms in the hydrocarbon group having 1 to 50 carbon atoms is preferably 1 to 30, more preferably 1 to 25, particularly 1 to 20. m is preferably an integer of 1 to 500, more preferably 1 to 100, particularly 1 to 50. n is preferably an integer of 1 to 100, more preferably 1 to 80, particularly 1 to 50.

An alkoxy-modified silicone represented by Formula (7) may for example be KF-851 and X-22-801B (trade name) produced by Shin-Etsu Chemical Co. Ltd.

A cyclic silicone may for example be a compound represented by Formula (8):

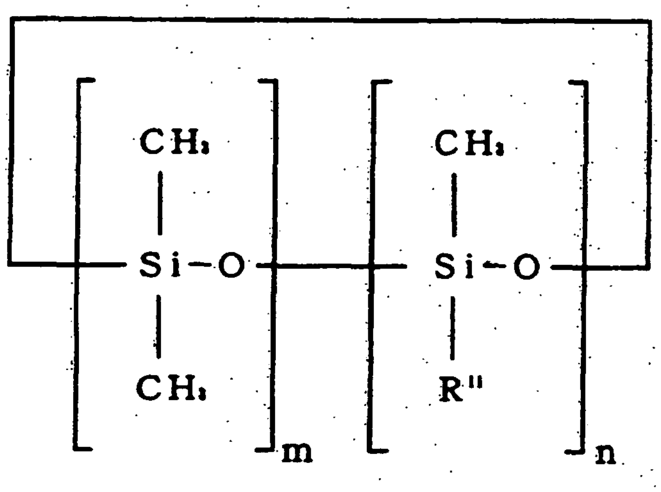

... (8)

wherein $R^{11}$ is a hydrocarbon group having 2 to 12 carbon atoms which may be same or different among repeating units, m is an integer of 1 or more, n is an integer of 0 or more, and m+n=3 to 10.

In Formula (8), $R^{11}$ may for example be a straight or branched saturated hydrocarbon group having 1 to 12 carbon atoms. The number of the carbon atoms in $R^{11}$ is preferably 2 to 10, more preferably 2 to 8, particularly 2 to 5. m is preferably an integer of 3 to 8, more preferably 4 to 8, particularly 4 to 6. n is preferably an integer of 0 to 7, more preferably 0 to 5, particularly 0 to 3. m+n is preferably 3 to 8, more preferably 4 to 8, particularly 4 to 6.

A cyclic silicone represented by Formula (8) may for example be SH244, SH344, SH245, DC345 and DC246 (trade name) produced by Dow Corning Toray Silicone Co. Ltd., as well as KF994, KF995 and KF9937 (trade name) produced by Shin-Etsu Chemical Co. Ltd.

The repeating units of the compounds represented by Formulae (3) and (5) to (8) may be of any type of the polymerization such as random polymerization and block polymerization.

The viscosity (dynamic viscosity) of a compound (S) at 25°C is preferably 1 to 5000 $mm^2/s$, more preferably 1 to 2000 $mm^2/s$, particularly 1 to 1000 $mm^2/s$. A preferred compound (S) is a dimethylpolysiloxane or polyether-modified silicone. The compound (S) can be employed alone or in combination.

A polysiloxane-carrying amphiphilic urethane resin of the invention is an amphiphilic urethane resin produced by reacting at least compounds (A) to (D) described above in the presence of a compound (S) described above. Such a polysiloxane-carrying amphiphilic urethane resin according to the invention can be produced using at least compounds (A) to (D) and (S) described above, by a method comprising a first step for producing an isocyanate group-containing prepolymer by reacting the compounds (A), (B) and (C) under an isocyanate group-excess condition and a second step for reacting said isocyanate group-containing prepolymer with the compound (D), wherein the compound (S) is allowed to exist in at least one of the first step and second step. In the production, the order of the reactions of the compounds (C) and (D) may be exchanged.

The weight ratio between a compound (S) and compounds (A), (B), (C) and (D), thus, (S)/((A)+(B)+(C)+(D)) is preferably 0.1/100 to 30/100, more preferably, 0.5/100 to 25/100, particularly 1/100 to 20/100.

The molar ratio between a compound (B) and compounds (A), (C) and (D), thus, (B)/((A)+(C)+(D)) is preferably 2.0/1.8 to 2.0/0.8, more preferably, 2.0/1.8 to 2.0/1.0, particularly 2.0/1.8 to 2.0/1.2.

The reactions in the first step and second step described above are conducted under the conditions employed ordinarily for producing a polyurethane in the presence of polymerization catalysts as appropriate. Such a polymerization

catalyst may be one employed ordinarily for producing an urethane resin. The polymerization catalyst may for example be a tertiary amine catalyst, organometal catalyst and the like. The tertiary amine catalyst may for example be [2.2.2] diazabicyclooctane (DABCO), tetramethylenediamine, N-methylmorpholine, diazabicycloundecene (DBU) and the like. The organometal catalyst may for example be dibutyltin dilaurate and the like.

For producing an amphiphilic urethane resin described above, the reactions in the first step and the second step may employ organic solvents as desired, and it is preferred for example to use an organic solvent capable of dissolving the both of compounds (A) to (D) and a resultant amphiphilic urethane resin. Such an organic solvent may for example be an amide such as N-methylpyrrolidone, dimethylformamide and dimethylacetamide, a ketone such as acetone and methylethylketone, an ester such as ethyl acetate, as well as cellosolve acetate and cellosolve ether.

After a second step, the reaction product from the second step is preferably mixed with water to perform a chain elongation reaction. After the second step, the reaction product from the step is mixed with basic water to perform the chain elongation reaction. Alternatively, the reaction product from the second step is preferably admixed with a basic compound and then combined with water to perform the chain elongation reaction. Among such procedures, the procedure in which after the second step the reaction product from the step is mixed with basic water to perform the chain elongation reaction is especially preferred. In the invention, it is preferred that the reactions in the first step and second step are performed in an organic solvent and then the reaction product from the second step is mixed with basic water to perform the chain elongation reaction consecutively in water. Such an embodiment involving the mixing of the reaction product from the second step with the basic water to perform the chain elongation reaction consecutively in water is preferred because it allows an amphiphilic urethane resin whose molecular weight is increased to be obtained readily. In the production method in this embodiment, it is preferable to select a production condition capable of yielding a reaction product from the second step which is a prepolymer containing an isocyanate group on its terminal.

The basic water described above means water containing a basic substance dissolved therein and exhibiting a basic nature, such as water containing triethylamine, triethanolamine, ammonia, potassium hydroxide, sodium hydroxide, 2-amino-2-methyl-1-propanol and the like dissolved therein.

A chain elongation reaction in the process for producing a polysiloxane-carrying amphiphilic urethane resin according to the invention may employ a chain elongation agent, and such a chain elongation agent serves to control the characteristics of the polysiloxane-carrying amphiphilic urethane resin as a final product. The chain elongation agent is a compound employed in a chain elongation reaction such as a low molecular weight polyol, amine, water and the like. Such a low molecular weight polyol may for example be a glycol such as ethylene glycol, propylene glycol, 1,4-butanediol, diethylene glycol, 1,6-hexanediol, spiroglycol, bis(β-hydroxyethoxy)benzene, xylylene glycol and the like, as well as a triol such as trimethylol propane, glycerin and the like. An amine mentioned above may for example be methylene (bis-o-chloroaniline) and the like.

In the invention, it is preferable to use as a polysiloxane-carrying amphiphilic urethane resin one having a structural unit derived from an alkylene oxide (hereinafter sometimes referred to as RO) in the resin structure for the purpose of improving the stability and the characteristics of a cosmetic formulation. The structural unit derived from RO may for example be an ethylene oxide (hereinafter sometimes referred to as EO) unit or a propylene oxide (hereinafter sometimes referred to as PO) unit, with the EO unit being preferred.

A compound having a structural unit derived from RO in its structure is not limited particularly provided that it is capable of introducing the structural unit derived from RO into the structure of a polysiloxane-carrying amphiphilic urethane resin.

A compound having a structural unit derived from RO in its structure is preferably a compound having at least one selected from a hydroxyl group, primary amino group and secondary amino group and a structural unit represented by Formula (1):

$$-(C_2H_4O)_p(C_3H_6O)_q- \qquad (1)$$

wherein p is an integer of 1 to 500 and q is an integer of 0 to 400.

Formula (1) wherein q is 0 gives a polymer of $C_2H_4O$ (polyoxyethylene), while that wherein q is not 0 gives a copolymer of $C_2H_4O$ with $C_3H_6O$. Such a copolymer may be a random copolymer or block copolymer.

In Formula (1), p is an integer of 1 to 500. Whether q is not 0 or q is 0, p is preferably 3 to 250, more preferably 3 to 120, particularly 3 to 50. q less than 1 leads to a too small amount of the EO units introduced into an amphiphilic urethane resin, resulting in a poor hydrophilicity, which makes a hair conditioner, for example, to which the amphiphilic urethane resin is applied poorly hydrophilic and poorly hair-washable. On the other hand, n exceeding 500 leads to a too high hydrophilicity of an amphiphilic urethane resin itself, resulting in an adverse effect on the moisture resistance and the like.

Also, the repeating number q of the PO units in Formula (1) is an integer of 0 to 400, preferably q=0. When q is not 0, it is preferred to select as q a number within the range of 3 to 200, more preferably 3 to 100. A particularly preferred number is 3 to 40. Whether q is not 0 or q is 0, p+q is preferably within the range from 3 to 300, more preferably

10 to 120, particularly 3 to 50.

Whether q is 0 or not, the weight ratio between the EO units and the PO units as EO units/PO units is within the range preferably from 10/0 to 2/8, more preferably from 10/0 to 3/7, particularly 10/0 to 4/6.

A compound (E) is preferably of both-terminal OH introduction type, both-terminal $NH_2$ introduction type, one-terminal OH introduction type and one-terminal $NH_2$ introduction type. When using a both-terminal OH introduction type or both-terminal $NH_2$ introduction type compound, an amphiphilic urethane resin having a structural unit represented by Formula (1) in its backbone is obtained. Also, when using a one-terminal OH introduction type or one-terminal $NH_2$ introduction type compound, an amphiphilic urethane resin having a structural unit represented by Formula (1) on its side chain or on its terminal is obtained.

The weight mean molecular weight of a compound (E) described above is preferably 200 to 20000, more preferably 200 to 5000, particularly 500 to 2000.

A compound (E) may for example be a polyethylene glycol (PEG), polyethylene polypropylene glycol, polyethylene polypropylene block copolymer and the like. Among those listed above, a polyethylene glycol is employed preferably. The compound (E) can be employed alone or in combination.

A polysiloxane-carrying amphiphilic urethane resin when a compound (E) is added is preferably an amphiphilic urethane resin produced by reacting at least compounds (A) to (E) described above in the presence of a compound (S) described above.

Such a resin can be produced by a method similar to that employed when a compound (E) is not added. Thus, it can be produced using at least compounds (A) to (E) and (S) described above, by a method comprising a first step for producing an isocyanate group-containing prepolymer by reacting the compounds (A), (B), (C) and (E) under an isocyanate group-excess condition and a second step for reacting said isocyanate group-containing prepolymer with the compound (D), wherein the compound (S) is allowed to exist in at least one of the first step and second step. It is also possible that the order of the reactions of the compounds (C) and (D) may be exchanged.

The weight ratio between a compound (S) and compounds (A), (B), (C), (D) and (E), thus, (S)/((A)+(B)+(C)+(D)+(E)) is preferably 0.1/100 to 30/100, more preferably, 0.5/100 to 25/100, particularly 1/100 to 20/100.

The molar ratio between a compound (B) and compounds (A), (C), (D) and (E), thus, (B)/((A)+(C)+(D)+(E)) is preferably 2.0/1.8 to 2.0/0.8, more preferably, 2.0/1.8 to 2.0/1.0, particularly 2.0/1.8 to 2.0/1.2.

The reactions in the first step and second step described above are conducted under the conditions employed ordinarily for producing a urethane in the presence of polymerization catalysts as appropriate similarly to the procedure employing no compound (E). The polymerization catalyst, organic solvent and chain elongation reaction are as discussed above. Also when adding a compound (E), the first step and the second step are conducted preferably in an organic solvent, with the chain elongation reaction performed in water being more preferred.

A polysiloxane-carrying amphiphilic urethane resin of the invention has a carboxyl group and a tertiary amino group in one molecule. The ratio of the carboxyl group and the tertiary amino group (number ratio between both functional groups) when represented as carboxyl group/tertiary amino group is preferably 1/50 to 50/1, more preferably 1/1 to 50/1, particularly 1/1 to 25/1. A ratio of the carboxyl group and the tertiary amino group in a polysiloxane-carrying amphiphilic urethane resin within the range from 1/50 to 50/1 gives a hair conditioner containing such a polysiloxane-carrying amphiphilic urethane resin capability to impart a further improved touch to the hair. When effecting a reaction, the molar ratio of a compound (C) and a compound (D), i.e., compound (C)/compound (D) is preferably 1/50 to 50/1, more preferably 1/1 to 50/1, particularly 1/1 to 25/1.

A polysiloxane-carrying amphiphilic urethane resin according to the invention is not one containing the polysiloxane chain of a polysiloxane compound in its backbone via a covalent bond but is one containing the polysiloxane chain as a result of restricting the polysiloxane compound by the backbone of the amphiphilic urethane resin or as a result of tangling the polysiloxane chain of the polysiloxane compound mechanically with the backbone of the amphiphilic urethane resin. Such a restricting or tangling structure is believed to become further complicated as the polymerization reaction of an amphiphilic urethane is further advanced, resulting in a difficulty in allowing the polysiloxane compound to be released from the resultant amphiphilic urethane resin.

Such a restricting or tangling state between the backbone of an amphiphilic urethane resin and a polysiloxane compound is referred here to as a state in which the backbone of the amphiphilic urethane resin is "carrying" the polysiloxane compound. The "carrying" has a meaning here which may vary depending on whether the amphiphilic urethane resin is in the form of an aqueous solution or aqueous dispersion. While the backbone of the amphiphilic urethane resin is usually in a straight chain structure, it may be in a branched chain structure or crosslinked structure, and it is understood that the polysiloxane chain is intercalated in the backbone of the amphiphilic urethane resin when the amphiphilic urethane resin is in the form of an "aqueous solution".

On the other hand, the amphiphilic urethane resin in the form of an "aqueous dispersion" is understood to be in a state where the amphiphilic urethane resin is present as a particle dispersed in water, the particle being restricted by the polysiloxane chain in various morphologies. In the first morphology, the polysiloxane chain is' enclosed entirely or partially in the particle. In the second morphology, a terminal of the polysiloxane chain is enclosed in the particle. In

the third morphology, the polysiloxane chain is deposited on the surface of the particle. Any of the first to third morphologies represents the "restricting" state, and mixture of the first to third morphologies also represents the "restricting" state.

As discussed above, the backbone of an amphiphilic urethane resin according to the invention carries a polysiloxane compound. As a result, the polysiloxane chain is considered to be difficult to be released from the amphiphilic urethane resin while it maintains relatively higher mobility.

The Production Examples of polysiloxane-carrying amphiphilic urethane resins according to the invention and other urethane resins for comparison are described below.

[Production Example 1: Polysiloxane-carrying amphiphilic urethane resin (A)]

A four-necked flask fitted with a stirrer, thermometer, nitrogen inlet and condenser was filled with 70 g of isophorone diisocyanate (IPDI), 63 g of a polypropylene glycol (PPG, weight mean molecular weight: 1000), 7 g of 1,4-cyclohexane dimethanol (CHDM), 8 g of a dimethylpolysiloxane (viscosity at 25˚C: 10 $mm^2/s$, SH200C-10cs (trade name) produced by Dow Corning Toray Silicone Co. Ltd.) and 20 g of dimethylol butanoic acid (DMBA) together with 50 g of ethyl acetate as a solvent, which were heated to 80˚C using an oil bath and reacted for 3 hours. Subsequently, 2 g of N-methyldiethanolamine (NMDEtA) and 60 g of ethyl acetate were further added and reacted at 80˚C for further 3 hours to obtain a prepolymer in which the isocyanate group was still remaining. After this prepolymer in which the isocyanate group was still remaining was cooled to 50˚C, it was dispersed in 700 g of water containing 10 g of potassium hydroxide by a high speed agitation, and then subjected to a chain elongation reaction at 50˚C for 3 hours to increase the molecular weight. From the resultant aqueous liquid, ethyl acetate was recovered to obtain an aqueous liquid of a polysiloxane-carrying amphiphilic urethane resin (A) containing substantially no solvent.

[Production Example 2: Polysiloxane-carrying amphiphilic urethane resin (B)]

Using the method similar to that in Production Example 1 except for using 8 g of a polyether-modified silicone (viscosity at 25˚C: 1600 $mm^2/s$, SH3775C (trade name) produced by Dow Corning Toray Silicone Co. Ltd.) instead of 8 g of the dimethylpolysiloxane employed in Production Example 1, an aqueous liquid of a polysiloxane-carrying amphiphilic urethane resin (B) was obtained.

[Production Example 3: Polysiloxane-carrying amphiphilic urethane resin (C)]

Using the method similar to that in Production Example 1 except for using 8 g of a cyclic silicone (viscosity at 25˚C: 4 $mm^2/s$, SH245 (trade name) produced by Dow Corning Toray Silicone Co. Ltd.) instead of 8 g of the dimethylpolysiloxane employed in Production Example 1, an aqueous liquid of a polysiloxane-carrying amphiphilic urethane resin (C) was obtained.

[Production Example 4: Polysiloxane-carrying amphiphilic urethane resin (D)]

Using the method similar to that in Production Example 1 except for using 8 g of a phenyl-modified silicone (viscosity at 25˚C: 22 $mm^2/s$, SH556 (trade name) produced by Dow Corning Toray Silicone Co. Ltd.) instead of 8 g of the dimethylpolysiloxane employed in Production Example 1, an aqueous liquid of a polysiloxane-carrying amphiphilic urethane resin (D) was obtained.

[Production Example 5: Polysiloxane-carrying amphiphilic urethane resin (E)]

Using the method similar to that in Production Example 1 except for using 8 g of an alkyl-modified silicone (viscosity at 25˚C: 500 $mm^2/s$, KF-412 (trade name) produced by Shin-Etsu Chemical Co. Ltd.) instead of 8 g of the dimethylpolysiloxane employed in Production Example 1, an aqueous liquid of a polysiloxane-carrying amphiphilic urethane resin (E) was obtained.

[Production Example 6: Polysiloxane-carrying amphiphilic urethane resin (F)]

Using the method similar to that in Production Example 1 except for using 8 g of an alkoxy-modified silicone (viscosity at 25˚C: 80 $mm^2/s$, KF-851 (trade name) produced by Shin-Etsu Chemical Co. Ltd.) instead of 8 g of the dimethylpolysiloxane employed in Production Example 1, an aqueous liquid of a polysiloxane-carrying amphiphilic urethane resin (F) was obtained.

[Production Example 7: Polysiloxane-carrying amphiphilic urethane resin (G)]

Using the method similar to that in Production Example 1 except for using 8 g of a dimethylpolysiloxane (viscosity at 25˚C: 10 $mm^2/s$, SH200C-10cs (trade name) produced by Dow Corning Toray Silicone Co. Ltd.) instead of 8 g of the dimethylpolysiloxane employed in Production Example 1, an aqueous liquid of a polysiloxane-carrying amphiphilic urethane resin (G) was obtained.

[Production Example 8: Polysiloxane-carrying amphiphilic urethane resin (H)]

Using the method similar to that in Production Example 1 except for using 20 g of a polyether-modified silicone (viscosity at 25˚C: 1600 $mm^2/s$, SH3775C (trade name) produced by Dow Corning Toray Silicone Co. Ltd.) instead of 8 g of the dimethylpolysiloxane employed in Production Example 1, an aqueous liquid of a polysiloxane-carrying amphiphilic urethane resin (H) was obtained.

[Production Example 9: Polysiloxane-carrying amphiphilic urethane resin (I)]

A four-necked flask fitted with a stirrer, thermometer, nitrogen inlet and condenser was filled with 70 g of isophorone diisocyanate (IPDI), 55 g of a polypropylene glycol (PPG, weight mean molecular weight: 1000), 8 g of a polyethylene glycol (PEG, weight mean molecular weight: 1000), 7 g of 1,4-cyclohexane dimethanol (CHDM), 8 g of a dimethylpolysiloxane (viscosity at 25˚C: 10 $mm^2/s$, SH200C-10cs (trade name) produced by Dow Corning Toray Silicone Co. Ltd.) and 20 g of dimethylol butanoic acid (DMBA) together with 50 g of ethyl acetate as a solvent, which were heated to 80˚C using an oil bath and reacted for 3 hours. Subsequently, 2 g of N-methyldiethanolamine (NMDEtA) and 60 g of ethyl acetate were further added and reacted at 80˚C for further 3 hours to obtain a prepolymer in which the isocyanate group was still remaining. After this prepolymer in which the isocyanate group was still remaining was cooled to 50˚C, it was dispersed in 700 g of water containing 10 g of potassium hydroxide by a high speed agitation, and then subjected to a chain elongation reaction at 50˚C for 3 hours to increase the molecular weight. From the resultant aqueous liquid, ethyl acetate was recovered to obtain an aqueous liquid of a polysiloxane-carrying amphiphilic urethane resin (I) containing substantially no solvent.

[Production Example 10: Polysiloxane-carrying amphiphilic urethane resin (J)]

Using the method similar to that in Production Example 9 except for using 8 g of a polyether-modified silicone (viscosity at 25˚C: 1600 $mm^2/s$, SH3775C (trade name) produced by Dow Corning Toray Silicone Co. Ltd.) instead of 8 g of the dimethylpolysiloxane employed in Production Example 9, an aqueous liquid of a polysiloxane-carrying amphiphilic urethane resin (J) was obtained.

[Production Example 11: Polysiloxane-carrying amphiphilic urethane resin (K)]

Using the method similar to that in Production Example 9 except for using 20 g of a dimethylpolysiloxane (viscosity at 25˚C: 10 $mm^2/s$, SH200C-10cs (trade name) produced by Dow Corning Toray Silicone Co. Ltd.) instead of 8 g of the dimethylpolysiloxane employed in Production Example 9, an aqueous liquid of a polysiloxane-carrying amphiphilic urethane resin (K) was obtained.

[Production Example 12: Polysiloxane-carrying amphiphilic urethane resin (L)]

Using the method similar to that in Production Example 9 except for using 20 g of a polyether-modified silicone (viscosity at 25˚C: 1600 $mm^2/s$, SH3775C (trade name) produced by Dow Corning Toray Silicone Co. Ltd.) instead of 8 g of the dimethylpolysiloxane employed in Production Example 9, an aqueous liquid of a polysiloxane-carrying amphiphilic urethane resin (L) was obtained.

[Comparative Production Example 1: Amphiphilic urethane resin (M)]

Using the method similar to that in Production Example 1 without using dimethylpolysiloxane used in Production Example 1, an aqueous liquid of an amphiphilic urethane resin (M) was obtained.

[Comparative Production Example 2: Amphiphilic urethane resin (N)]

A four-necked flask fitted with a stirrer, thermometer, nitrogen inlet and condenser was filled with 70 g of

isophorone diisocyanate (IPDI), 63 g of a polypropylene glycol (PPG, weight mean molecular weight: 1000), 7 g of 1,4-cyclohexane dimethanol (CHDM) and 20 g of dimethylol butanoic acid (DMBA) together with 50 g of ethyl acetate as a solvent, which were heated to 80˚C using an oil bath and reacted for 3 hours. Subsequently, 2 g of N-methyldiethanolamine (NMDEtA) and 60 g of ethyl acetate were further added and reacted at 80˚C for further 3 hours to obtain a prepolymer in which the isocyanate group was still remaining. After this prepolymer in which the isocyanate group was still remaining was cooled to 50˚C, it was dispersed in 700 g of water containing 10 g of potassium hydroxide by a high speed agitation, and then subjected to a chain elongation reaction at 50˚C for 3 hours to increase the molecular weight. From the resultant aqueous liquid, ethyl acetate was recovered to obtain an aqueous liquid of an amphiphilic urethane resin containing substantially no solvent, and then 8 g of a dimethylpolysiloxane (viscosity at 25˚C: 10 $mm^2/s$, SH200C-10cs (trade name) produced by Dow Corning Toray Silicone Co. Ltd.) was added to obtain an aqueous liquid of an amphiphilic urethane resin (N).

[Comparative Production Example 3: Amphiphilic urethane resin (O)]

Using the method similar to that in Comparative Production Example 2 except for using 8 g of a polyether-modified silicone (viscosity at 25˚C: 1600 $mm^2/s$, SH3775C (trade name) produced by Dow Corning Toray Silicone Co. Ltd.) instead of 8 g of the dimethylpolysiloxane employed in Comparative Production Example 2, an aqueous liquid of an amphiphilic urethane resin (O) was obtained.

[Comparative Production Example 4: Amphiphilic urethane resin (P)]

Using the method similar to that in Comparative Production Example 2 except for using 8 g of a cyclic silicone (viscosity at 25˚C: 4 $mm^2/s$, SH245 (trade name) produced by Dow Corning Toray Silicone Co. Ltd.) instead of 8 g of the dimethylpolysiloxane employed in Comparative Production Example 2, an aqueous liquid of an amphiphilic urethane resin (P) was obtained.

[Comparative Production Example 5: Amphiphilic urethane resin (Q)]

Using the method similar to that in Comparative Production Example 2 except for using 8 g of a phenyl-modified silicone (viscosity at 25˚C: 22 $mm^2/s$, SH556 (trade name) produced by Dow Corning Toray Silicone Co. Ltd.) instead of 8 g of the dimethylpolysiloxane employed in Comparative Production Example 2, an aqueous liquid of an amphiphilic urethane resin (Q) was obtained.

[Comparative Production Example 6: Amphiphilic urethane resin (R)]

Using the method similar to that in Comparative Production Example 2 except for using 8 g of a alkyl-modified silicone (viscosity at 25˚C: 500 $mm^2/s$, KF-412 (trade name) produced by Shin-Etsu Chemical Co. Ltd.) instead of 8 g of the dimethylpolysiloxane employed in Comparative Production Example 2, an aqueous liquid of an amphiphilic urethane resin (R) was obtained.

[Comparative Production Example 7: Amphiphilic urethane resin (S)]

Using the method similar to that in Comparative Production Example 2 except for using 8 g of a alkoxy-modified silicone (viscosity at 25˚C: 80 $mm^2/s$, KF-851 (trade name) produced by Shin-Etsu Chemical Co. Ltd.) instead of 8 g of the dimethylpolysiloxane employed in Comparative Production Example 2, an aqueous liquid of an amphiphilic urethane resin (S) was obtained.

[Comparative Production Example 8: Amphiphilic urethane resin (T)]

Using the method similar to that in Production Example 1 except for using 8 g of a dimethylpolysiloxanediol (both-terminal OH introduction type, viscosity at 25˚C: 62 $mm^2/s$, KF-6002 (trade name) produced by Shin-Etsu Chemical Co. Ltd.) and 55 g of a polypropylene glycol (PPG, weight mean molecular weight: 1000) instead of 8 g of the dimethylpolysiloxane and 63 g of the polypropylene glycol (PPG, weight mean molecular weight: 1000), respectively, employed in Production Example 1, an aqueous liquid of an amphiphilic urethane resin (T) was obtained.

[Comparative Production Example 9: Amphiphilic urethane resin (U)]

Using the method similar to that in Production Example 1 except for using 8 g of a dimethylpolysiloxanediol

(both-terminal OH introduction type, viscosity at 25˚C: 88 $mm^2/s$, X-22-176B (trade name) produced by Shin-Etsu Chemical Co. Ltd.) and 55 g of a polypropylene glycol (PPG, weight mean molecular weight: 1000) instead of 8 g of the dimethylpolysiloxane and 63 g of the polypropylene glycol (PPG, weight mean molecular weight: 1000), respectively, employed in Production Example 1, an aqueous liquid of an amphiphilic urethane resin (U) was obtained.

Cosmetic formulations containing polysiloxane-carrying amphiphilic urethane resins are discussed below.

In a cosmetic formulation of the invention, it is preferred to use a polysiloxane-carrying amphiphilic urethane resin as an aqueous liquid, and a polysiloxane-carrying amphiphilic urethane resin according to the invention preferably forms an aqueous liquid when being mixed with water. In the invention, such an aqueous liquid means an aqueous solution state in which a polysiloxane-carrying amphiphilic urethane resin is dissolved completely in water as well as an aqueous dispersion state and/or aqueous suspension state in which a polysiloxane-carrying amphiphilic urethane resin is dispersed and/or suspended in water. Nevertheless, it is also acceptable to use a resin component of a polysiloxane-carrying amphiphilic urethane resin obtained by substantially removing solvents such as water. An aqueous liquid of a polysiloxane-carrying amphiphilic urethane resin described above may be imparted with a crosslinking ability by adding a crosslinking agent such as a silane coupling agent. Various additives can also be added for obtaining the storage stability, and protective colloidal agents, antibacterial agents and antifungal agents may be mentioned.

A cosmetic formulation of the invention can be obtained in a standard manner by incorporating a polysiloxane-carrying amphiphilic urethane resin described above as a component of the cosmetic formulation. The invention is advantageous especially in obtaining an excellent cosmetic formulation capable of exerting the functional coating characteristics of a polysiloxane-carrying amphiphilic urethane resin. Such a cosmetic formulation is not limited particularly, and the invention can be applied widely to hair formulations such as hair dressing formulations including hair dressing foams, hair dressing gels, hair dressing aerosol sprays, hair dressing pump sprays as well as hair conditioners, makeup formulations such as mascaras, eyeliners, nail polishes, foundations and lip colors, dermal external formulations such as facial packs and masks, shaving aids, creams, milky lotions, lotions, essences (beauty essences) and the like, fragrances, body formulations and the like.

The form of a cosmetic formulation may also vary widely, including solution systems, solubilized systems, emulsion systems, foams, powder systems, powder dispersion systems, oil systems, gel systems, ointment systems, aerosol systems, spray systems, pump spray systems, water-oil two-layer systems, water-oil-powder three-layer systems and the like.

While the amount of a polysiloxane-carrying amphiphilic urethane resin described above in the invention may vary depending on the types of the cosmetic formulations, it is preferably 0.1 to 25.0% by weight based on the total amount of the cosmetic formulation. For example, the amount when used as a film-forming agent in a hair formulation is preferably 0.1 to 10.0% by weight based on the total amount of the cosmetic formulation, more preferably 0.5 to 0.8% by weight. The amount when used in a makeup formulation such as mascara, eyeliner, nail polish, foundation, lip color and the like is preferably 0.1 to 25.0% by weight based on the total amount of the cosmetic formulation. The amount in a facial pack or mask is preferably 0.1 to 25.0% by weight based on the total amount of the cosmetic formulation. The amount in a dermal external formulation such as a shaving formulation, cream, milky lotion, lotion, essence (beauty essence) and the like is preferably 0.1 to 15.0% by weight based on the total amount of the cosmetic formulation.

In addition to the components described above, other auxiliary components employed usually in cosmetic and pharmaceutical formulations may also be added to an inventive cosmetic formulation as long as they do not affect the invention adversely. For example, oil components, powder components, surfactants, humectants, water-soluble polymers, thickening agent, coatings, UV absorbers, metal ion sequestering agents, saccharides, amino acids, organic amines, pH modifiers, skin nutrients, vitamins, antioxidants, fragrances and water can be mentioned.

The invention is further detailed in Examples shown below. An amount indicated is a % by weight. A % is a % by weight unless otherwise specified. Prior to the description of Examples, the tests for evaluating the effects of the invention are described below.

(Elasticity: Curl memory method)

A black virgin hair (20 cm in length, 4 g in weight) was coated with 0.5 g of a prepared styling moose, and 5 curls were produced per sample and dried at 50˚C for 1 hour. The length of each curled hair strand was measured and recorded as an initial value (c). Then a 60g load was applied to the tip of the hair over a period of 15 minutes, after which the load was removed and the scale was read at the hair tip point (d). According to the following equation, the curl memory value was calculated.

$$\text{Curl memory value } (\%) = \{(20-d)/(20-c)\} \times 100$$

A curl memory value closer to 100% indicates a higher % curl maintenance and higher elasticity. The evaluation criteria are shown below.
⊙ : 90% or higher
○ : 70 to less than 90%
Δ : 50 to less than 70%
× : Less than 50%

(Flaking)

A black virgin hair (20 cm in length, 4 g in weight) was coated with 0.5 g of a prepared styling moose, dried at 50˚C for 1 hour, and then allowed to stand in a thermostat chamber at 25˚C and 60% relative humidity over a period of 30 minutes. This hair strand was subjected to a 5-time combing and the resultant flaking was examined visually.
The evaluation criteria are shown below.
⊙ : Absolutely no flaking
○: No flaking
Δ : Slight flaking
× : Flaking

(Stability)

30 samples of a styling moose were prepared in transparent containers, subjected to a thermal cycle test (50˚C, to -10˚C, 2 cycle/day, 1 month), examined visually by a trained expert engineer for any separation, creaming or aggregation, and evaluated in accordance with the following criteria.
⊙ : Zero samples exhibited separation, creaming, aggregation and the like.
○: One sample exhibited separation, creaming, aggregation and the like.
Δ : Two samples exhibited separation, creaming, aggregation and the like.
× : Three of more samples exhibited separation, creaming, aggregation and the like.

(Examples 1 to 6)

First, the characteristics of an inventive cosmetic formulation were demonstrated by conducting an evaluation test using a styling moose as a hair formulation. Styling mousses of Examples 1 to 6 were produced using the formulations shown in Table 1 and examined for the elasticity, flaking and emulsion stability. The results are also indicated in Table 1. The polysiloxane-carrying amphiphilic urethane was added as an aqueous liquid.

(Production method)

For obtaining an emulsion part, the component (1) was added to the components (2), (3), (4) and a part of the component (16) and the mixture was emulsified using a homomixer. Then a part of the component (15) was added to form the emulsion part. An ethanol part was obtained by dissolving the components (5), (12) to (14) by stirring. For obtaining an aqueous part, the remainder of the component (16), components (6) to (11) and the remainder of the component (15) were admixed and stirred until uniform. These three parts were mixed appropriately to obtain a moose stock solution. 92 Parts of the resultant moose stock solution was filled in an aerosol canister, to which a valve was fitted and 8 parts of liquefied petroleum gas (LPG) was charged, whereby obtaining each styling moose.

Table 1

| | | Example | | | | | |
|---|---|---|---|---|---|---|---|
| | | 1 | 2 | 3 | 4 | 5 | 6 |
| 1 | Dimethylpolysiloxane (6mPa·s) | 5.0 | 5.0 | 5.0 | 5.0 | 5.0 | 5.0 |
| 2 | 1,3-Butylene glycol | 5.0 | 5.0 | 5.0 | 5.0 | 5.0 | 5.0 |
| 3 | Glycerin | 5.0 | 5.0 | 5.0 | 5.0 | 5.0 | 5.0 |
| 4 | Polyoxyetylene hardened caster oil (40EO) | 2.0 | 2.0 | 2.0 | 2.0 | 2.0 | 2.0 |
| 5 | Ethanol | 10.0 | 10.0 | 10.0 | 10.0 | 10.0 | 10.0 |

(continued)

| | | Example | | | | | |
|---|---|---|---|---|---|---|---|
| | | 1 | 2 | 3 | 4 | 5 | 6 |
| 6 | Polysiloxane-carrying amphiphilic urethane resin (A) (resin content 20%) | 15.0 | - | - | - | - | - |
| 7 | Polysiloxane-carrying amphiphilic urethane resin (B) (resin content 20%) | - | 15.0 | - | - | - | - |
| 8 | Polysiloxane-carrying amphiphilic urethane resin (C) (resin content 20%) | - | - | 15.0 | - | - | - |
| 9 | Polysiloxane-carrying amphiphilic urethane resin (D) (resin content 20%) | - | - | - | 15.0 | - | - |
| 10 | Polysiloxane-carrying amphiphilic urethane resin (E) (resin content 20%) | - | - | - | - | 15.0 | - |
| 11 | Polysiloxane-carrying amphiphilic urethane resin (F) (resin content 20%) | - | - | - | - | - | 15.0 |
| 12 | Special grade lauric acid diethanol amide (1:1 type) | 0.2 | 0.2 | 0.2 | 0.2 | 0.2 | 0.2 |
| 13 | Alkyltrimethylammonium chloride | 0.1 | 0.1 | 0.1 | 0.1 | 0.1 | 0.1 |
| 14 | Paraben | 0.1 | 0.1 | 0.1 | 0.1 | 0.1 | 0.1 |
| 15 | Polyoxyethylene lauryl ether (12EO) | 0.4 | 0.4 | 0.4 | 0.4 | 0.4 | 0.4 |
| 16 | Ion exchange water | bal. | bal. | bal. | bal. | bal. | bal. |
| Elasticity | | ◎ | ◎ | ◎ | ○ | ◎ | ○ |
| Flaking | | ◎ | ◎ | ○ | ◎ | ○ | ○ |
| Stability | | ○ | ◎ | ○ | ○ | ○ | ○ |

As evident from Examples 1 to 6 shown in Table 1, each of the styling mousses of Examples 1 to 6 which contained each of the polysiloxane-carrying amphiphilic urethane resins (A) to (F) according to the invention exhibited excellent results with regard to all of the elasticity, flaking and stability.

(Examples 7 to 12)

Using the formulations shown in Table 2, styling mousses of Examples 7 to 12 were produced by the method similar to that in Table 1 and examined for the elasticity, flaking and emulsion stability. The results are included in Table 2. Each polysiloxane-carrying amphiphilic urethane resin was added in the form of an aqueous liquid similarly to Table 1.

Table 2

| | | Example | | | | | |
|---|---|---|---|---|---|---|---|
| | | 7 | 8 | 9 | 10 | 11 | 12 |
| 1 | Dimethylpolysiloxane (6mPa·s) | 5.0 | 5.0 | 5.0 | 5.0 | 5.0 | 5.0 |
| 2 | 1,3-Butylene glycol | 5.0 | 5.0 | 5.0 | 5.0 | 5.0 | 5.0 |
| 3 | Glycerin | 5.0 | 5.0 | 5.0 | 5.0 | 5.0 | 5.0 |
| 4 | Polyoxyetylene hardened caster oil (40EO) | 2.0 | 2.0 | 2.0 | 2.0 | 2.0 | 2.0 |
| 5 | Ethanol | 10.0 | 10.0 | 10.0 | 10.0 | 10.0 | 10.0 |
| 6 | Polysiloxane-carrying amphiphilic urethane resin (G) (resin content 20%) | 15.0 | - | - | - | - | - |
| 7 | Polysiloxane-carrying amphiphilic urethane resin (H) (resin content 20%) | - | 15.0 | - | - | - | - |

(continued)

| | | Example | | | | | |
|---|---|---|---|---|---|---|---|
| | | 7 | 8 | 9 | 10 | 11 | 12 |
| 8 | Polysiloxane-carrying amphiphilic urethane resin (I) (resin content 20%) | - | - | 15.0 | - | - | - |
| 9 | Polysiloxane-carrying amphiphilic urethane resin (J) (resin content 20%) | - | - | - | 15.0 | - | - |
| 10 | Polysiloxane-carrying amphiphilic urethane resin (K) (resin content 20%) | - | - | - | - | 15.0 | - |
| 11 | Polysiloxane-carrying amphiphilic urethane resin (L) (resin content 20%) | - | - | - | - | - | 15.0 |
| 12 | Special grade lauric acid diethanol amide (1:1 type) | 0.2 | 0.2 | 0.2 | 0.2 | 0.2 | 0.2 |
| 13 | Alkyltrimethylammonium chloride | 0.1 | 0.1 | 0.1 | 0.1 | 0.1 | 0.1 |
| 14 | Paraben | 0.1 | 0.1 | 0.1 | 0.1 | 0.1 | 0.1 |
| 15 | Polyoxyethylene lauryl ether (12EO) | 0.4 | 0.4 | 0.4 | 0.4 | 0.4 | 0.4 |
| 16 | Ion exchange water | Bal. | Bal. | Bal. | Bal. | Bal. | Bal. |
| Elasticity | | ◎ | ◎ | ◎ | ◎ | ◎ | ◎ |
| Flaking | | ○ | ◎ | ◎ | ◎ | ○ | ◎ |
| Stability | | ○ | ◎ | ○ | ◎ | ○ | ◎ |

As evident from Examples 7 to 12 shown in Table 2, each of the styling mousses of Examples 7 to 12 which contained each of the polysiloxane-carrying amphiphilic urethane resins (G) to (L) according to the invention exhibited excellent results with regard to all of the elasticity, flaking and stability.

(Comparatives 1 to 5)

Using the formulations shown in Table 3, styling mousses of Comparative 1 to 5 were produced and examined for the elasticity, flaking and emulsion stability. The results are included in Table 3. Each amphiphilic urethane resin was added in the form of an aqueous liquid.

(Production method)

For obtaining an emulsion part, the component (1) was added to the components (2), (3), (4), a part of the component (15) and the mixture was emulsifying using a homomixer. Then a part of the component (14) was added to form the emulsion part. An ethanol part was obtained by dissolving the components (5), (11) to (13) by stirring. For obtaining an aqueous part, the remainder of the component (15), components (6) to (10) and the remainder of the component (14) were admixed and stirred until uniform. These three parts were mixed appropriately to obtain a moose stock solution. 92 Parts of the resultant moose stock solution was filled in an aerosol canister, to which a valve was fitted and 8 parts of liquefied petroleum gas (LPG) was charged, whereby obtaining each styling moose.

Table 3

| | | Comparative | | | | |
|---|---|---|---|---|---|---|
| | | 1 | 2 | 3 | 4 | 5 |
| 1 | Dimethylpolysiloxane (6mPa·s) | 5.0 | 5.0 | 5.0 | 5.0 | 5.0 |
| 2 | 1,3-Butylene glycol | 5.0 | 5.0 | 5.0 | 5.0 | 5.0 |
| 3 | Glycerin | 5.0 | 5.0 | 5.0 | 5.0 | 5.0 |
| 4 | Polyoxyetylene hardened caster oil (40EO) | 2.0 | 2.0 | 2.0 | 2.0 | 2.0 |
| 5 | Ethanol | 10.0 | 10.0 | 10.0 | 10.0 | 10.0 |

(continued)

| | | Comparative | | | | |
|---|---|---|---|---|---|---|
| | | 1 | 2 | 3 | 4 | 5 |
| 6 | Amphiphilic urethane resin (M) (resin content 20%) | 15.0 | - | - | - | - |
| 7 | Amphiphilic urethane resin (N) (resin content 20%) | - | 15.0 | - | - | - |
| 8 | Amphiphilic urethane resin (O) (resin content 20%) | - | - | 15.0 | - | - |
| 9 | Amphiphilic urethane resin (P) (resin content 20%) | - | - | - | 15.0 | - |
| 10 | Amphiphilic urethane resin (Q) (resin content 20%) | - | - | - | - | 15.0 |
| 11 | Special grade lauric acid diethanol amide (1:1 type) | 0.2 | 0.2 | 0.2 | 0.2 | 0.2 |
| 12 | Alkyltrimethylammonium chloride | 0.1 | 0.1 | 0.1 | 0.1 | 0.1 |
| 13 | Paraben | 0.1 | 0.1 | 0.1 | 0.1 | 0.1 |
| 14 | Polyoxyethylene lauryl ether (12EO) | 0.4 | 0.4 | 0.4 | 0.4 | 0.4 |
| 15 | Ion exchange water | Bal. | Bal. | Bal. | Bal. | Bal. |
| Elasticity | | Δ | Δ | Δ | ○ | Δ |
| Flaking | | ○ | ○ | Δ | × | × |
| Stability | | × | × | × | × | × |

As evident from Comparatives 1 to 5 in Table 3, each of the styling mousses of Comparatives 1 to 5 which contained each of the amphiphilic urethane resins (M) to (Q) departing from the scope of the invention was not satisfactory with regard to all of the elasticity, flaking and stability.

(Comparative 6 to 10)

Using the formulations shown in Table 4, styling mousses of Comparatives 6 to 10 were produced by the method similar to that in Table 3 and examined for the elasticity, flaking and emulsion stability. The results are included in Table 4. Each amphiphilic urethane resin added was in the form of an aqueous liquid similarly to Table 3.

Table 4

| | | Comparative | | | | |
|---|---|---|---|---|---|---|
| | | 6 | 7 | 8 | 9 | 10 |
| 1 | Dimethylpolysiloxane (6mPa·s) | 5.0 | 5.0 | 5.0 | 5.0 | 5.0 |
| 2 | 1,3-Butylene glycol | 5.0 | 5.0 | 5.0 | 5.0 | 5.0 |
| 3 | Glycerin | 5.0 | 5.0 | 5.0 | 5.0 | 5.0 |
| 4 | Polyoxyetylene hardened caster oil (40EO) | 2.0 | 2.0 | 2.0 | 2.0 | 2.0 |
| 5 | Ethanol | 10.0 | 10.0 | 10.0 | 10.0 | 10.0 |
| 6 | Amphiphilic urethane resin (R) (resin content 20%) | 15.0 | - | - | - | - |
| 7 | Amphiphilic urethane resin (S) (resin content 20%) | - | 15.0 | - | - | - |
| 8 | Amphiphilic urethane resin (T) (resin content 20%) | - | - | 15.0 | - | - |
| 9 | Amphiphilic urethane resin (U) (resin content 20%) | - | - | - | 15.0 | - |
| 10 | Amphiphilic polymer (resin content 30%)(Note) | - | - | - | - | 15.0 |
| 11 | Special grade lauric acid diethanol amide (1:1 type) | 0.2 | 0.2 | 0.2 | 0.2 | 0.2 |
| 12 | Alkyltrimethylammonium chloride | 0.1 | 0.1 | 0.1 | 0.1 | 0.1 |
| 13 | Paraben | 0.1 | 0.1 | 0.1 | 0.1 | 0.1 |

(continued)

| | | Comparative | | | | |
|---|---|---|---|---|---|---|
| | | 6 | 7 | 8 | 9 | 10 |
| 14 | Polyoxyethylene lauryl ether (12EO) | 0.4 | 0.4 | 0.4 | 0.4 | 0.4 |
| 15 | Ion exchange water | Bal. | Bal. | Bal. | Bal. | Bal. |
| Elasticity | | Δ | Δ | ○ | ○ | × |
| Flaking | | × | × | × | × | ○ |
| Stability | | × | × | × | × | Δ |
| (Note) *YUKAFOAMER* SM produced by Mitsubishi Chemical Co. Ltd. (Solution of a copolymer of N-methacryloyloxy-N,N-dimethylammonium-$\alpha$-N-methylcarboxybetaine/methacylic acid alkyl ester in ethanol) | | | | | | |

As evident from Comparatives 6 to 10 in Table 4, each of the styling mousses of Comparatives 6 to 9 which contained each of the amphiphilic urethane resins (R) to (U) departing from the scope of the invention was not satisfactory with regard to all of the elasticity, flaking and stability. Similarly, Comparative 10 containing an amphiphilic polymer which was not an amphiphilic urethane resin was not satisfactory with regard to all of the elasticity, flaking and stability.

Examples of the invention are further described below. Each polysiloxane-carrying amphiphilic urethane resin incorporated was an aqueous liquid. While the evaluation tests in the following descriptions were not detailed individually, they allowed the characteristics of each polysiloxane-carrying amphiphilic urethane resin of the invention to be exerted sufficiently.

[Example 13] Facial pack (jelly peel-off type)

| Ingredient | | Amount (% by wt) |
|---|---|---|
| (1) | Polyvinyl alcohol | 10.0 |
| (2) | Polysiloxane-carrying amphiphilic urethane resin (A) (20% aqueous liquid) | 2.5 (0.5% as resin) |
| (3) | Polysiloxane-carrying amphiphilic urethane resin (J) (20% aqueous liquid) | 2.5 (0.5% as resin) |
| (4) | Carboxymethyl celllulose | 5.0 |
| (5) | 1,3-Butylene glycol | 5.0 |
| (6) | Ethanol | 12.0 |
| (7) | Fragrance | Appropriate |
| (8) | Methylparaben | 0.2 |
| (9) | Buffer (citric acid, Na citrate) | Appropriate |
| (10) | Polyoxyethylene oleyl alcohol | 0.5 |
| (11) | Ion exchange water | Balance |

(Production method)

The components (9), (5), (2) and (3) were added to the component (11) and the mixture was heated at 70 to 80˚C. Then the components (4) and (1) were added and dissolved by stirring. The components (7), (8) and (10) were added to the component (6) and dissolved, and then the mixture was deaerated, filtered and cooled. The resultant product imparted a watery and smooth touch to the skin together with desirable suppleness.

[Example 14] O/W Emulsion foundation

| Ingredient | | Amount (% by wt) |
|---|---|---|
| (1) | Talc | 3.0 |

(continued)

| Ingredient | | Amount (% by wt) |
|---|---|---|
| (2) | Titanium dioxide | 5.0 |
| (3) | Red ocher | 0.5 |
| (4) | Iron oxide yellow | 1.4 |
| (5) | Iron oxide black | 0.1 |
| (6) | Bentonite | 0.5 |
| (7) | Polyoxyethylene sorbitan monostearate | 0.9 |
| (8) | Triethanolamine | 1.0 |
| (9) | Propylene glycol | 10.0 |
| (10) | Polysiloxane-carrying amphiphilic urethane resin (B) (20% aqueous liquid) | 10.0 (0.5% as resin) |
| (11) | Ion exchange water | Balance |
| (12) | Stearic acid | 2.2 |
| (13) | Isohexadecyl alcohol | 7.0 |
| (14) | Glycerin monostearate | 2.0 |
| (15) | Liquid lanolin | 2.0 |
| (16) | Liquid paraffin | 8.0 |
| (17) | Ethylparaben | 0.1 |
| (18) | Fragrance | Appropriate |

(Production method)

The component (6) dispersed in the component (9) was added to the component (11), treated using a homomixer at 70˚C, combined with the remaining aqueous phase components (7), (8) and (10), and stirred thoroughly. To this, the powder part which had been mixed and pulverized thoroughly was added, and treated using a homomixer at 70˚C. Then the liquid phase components (12) to (16) and the component (17) which had been heated and dissolved at 70 to 80˚C were added in portions, and treated using a homomixer at 70˚C. The mixture was cooled by stirring to 45˚C, at which the component (18) was added, and then the mixture was cooled to room temperature. Finally, the mixture was deaerated and filled in a container. The resultant emulsion foundation was watery and smooth, and gave a long-lasting makeup.

[Example 15] Eyeliner

| Ingredient | | Amount (% by wt) |
|---|---|---|
| (1) | Iron oxide black | 14.0 |
| (2) | Polysiloxane-carrying amphiphilic urethane resin (C) (20% aqueous liquid) | 45.0 (0.9% as resin) |
| (3) | Glycerin | 5.0 |
| (4) | Polyoxyethylene sorbitan monooleate | 1.0 |
| (5) | Carboxymethyl cellulose | 1.5 |
| (6) | Acetyltributyl citrate | 1.0 |
| (7) | Ion exchange water | Balance |
| (8) | Methylparaben | 0.1 |
| (9) | Fragrance | Appropriate |

(Production method)

The components (2), (3) and (4) were added to the component (7), heated and dissolved, and then combined with the component (1), and treated using a colloid mill (pigment part). The remaining components were added and the mixture was heated at 70˚C. To this mixture, the pigment part was added and dispersed uniformly using a homomixer. The resultant eyeliner exhibited suitable elasticity, and could be used satisfactorily.

[Example 16] Skin lotion

| Ingredient | | Amount (% by wt) |
|---|---|---|
| (1) | 1,3-Butylene glycol | 6.0 |
| (2) | Glycerin | 5.0 |
| (3) | Polyethylene glycol 400 | 3.0 |
| (4) | Olive oil | 0.5 |
| (5) | Polyoxyethylene(20)sorbitan monostearate | 1.5 |
| (6) | Polyoxyethylene(5)oleyl alcohol ether | 0.3 |
| (7) | Ethanol | 10.0 |
| (8) | Fragrance | Appropriate |
| (9) | Colorant | Appropriate |
| (10) | Phenoxyethanol | Appropriate |
| (11) | Citric acid | Appropriate |
| (12) | Sodium citrate | Appropriate |
| (13) | Anti-yellowing agent | Appropriate |
| (14) | Ion exchange water | Balance |
| (15) | Polysiloxane-carrying amphiphilic urethane resin (D) (20% aqueous liquid) | 0.5 (0.1% as resin) |
| (16) | Polysiloxane-carrying amphiphilic urethane resin (L) (20% aqueous liquid) | 0.5 (0.1% as resin) |

(Production method)

The components (1), (2), (3), (11), (12), (15) and (16) were added to the component (14) and dissolved at room temperature. On the other hand, the components (4), (5), (6), (10) and (8) were added to the component (7), and dissolved at room temperature. This alcohol phase was added to the aqueous phase described above, to which the components (9) and (13) were added to prepare a microemulsion. The resultant skin lotion was watery and smooth and imparted suppleness to the skin.

[Example 17] Milky lotion

| Ingredient | | Amount (% by wt) |
|---|---|---|
| (1) | Cetyl alcohol | 1.0 |
| (2) | Beeswax | 0.5 |
| (3) | Petrolatum | 2.0 |
| (4) | Squalane | 6.0 |
| (5) | Dimethylpolysiloxane (viscosity 20MPa·S) | 2.0 |
| (6) | Ethanol | 4.0 |
| (7) | Glycerin | 4.0 |
| (8) | 1,3-Buthylene glycol | 4.0 |
| (9) | Polyoxyethylene(10) monooleate | 1.0 |
| (10) | Glycerol monostearate | 1.0 |
| (11) | Quince seed extract (5% aqueous solution) | 10.0 |
| (12) | Polysiloxane-carrying amphiphilic urethane resin (E) (20% aqueous liquid) | 10.0 (2% as resin) |
| (13) | Methylparaben | Appropriate |
| (14) | Colorant (dye) | Appropriate |
| (15) | Fragrance | Appropriate |

(continued)

| | Ingredient | Amount (% by wt) |
|---|---|---|
| (16) | Ion exchange water | Balance |

(Production method)

The components (7), (8), (12) and (14) were added to the component (16), and heated at 70˚C. The components (1) to (5) were combined with the components (9) and (10), and heated at 70˚C. This was added to an aqueous phase and pre-emulsified. To this, the components (11) and (13) dissolved in the component (6) were added, and the emulsion particle was made uniform using a homomixer, and then the mixture was deaerated, filtered, combined with the component (15) and then cooled. The resultant milky lotion was watery and smooth, and imparted elasticity to the skin.

[Example 18] Cream

| | Ingredient | Amount (% by wt) |
|---|---|---|
| (1) | Stearyl alcohol | 6.0 |
| (2) | Stearic acid | 2.0 |
| (3) | Hydrogenated lanolin | 4.0 |
| (4) | Squalane | 9.0 |
| (5) | Octyl dodecanol | 10.0 |
| (6) | 1,3-Butylene glycol | 6.0 |
| (7) | Polyethylene glycol 1500 | 4.0 |
| (8) | Polysiloxane-carrying amphiphilic urethane resin (F) (20% aqueous liquid) | 3.0 (0.6% as resin) |
| (9) | Polyoxyethylene(25) cetyl alcohol ether | 3.0 |
| (10) | Glycerin monostearate | 2.0 |
| (11) | Methylparaben | Appropriate |
| (12) | Vitamin E | Appropriate |
| (13) | Fragrance | Appropriate |
| (14) | Ion exchange water | Balance |

(Production method)

The components (6), (7) and (8) were added to the component (14), and heated at 70˚C. The components (1) to (5) were heated and dissolved, and then combined with the components (9), (10), (11), (12) and (13), and adjusted at 70˚C. This was combined with the aqueous phase described above, and the emulsion particle was made uniform using a homomixer, and then the mixture was deaerated, filtered and cooled. The resultant cream was watery and smooth, and imparted elasticity to the skin.

[Example 19] Whitening essence

| | Ingredient | Amount (% by wt) |
|---|---|---|
| (1) | Dipropylene glycol | 5.0 |
| (2) | Polyethylene glycol 400 | 5.0 |
| (3) | Ethanol | 10.0 |
| (4) | Carboxyvinyl polymer | 0.3 |
| (5) | Sodium alginate | 0.3 |
| (6) | Potassium hydroxide | 0.15 |
| (7) | Polyoxyethylene sorbitan monostearate | 1.0 |
| (8) | Sorbitan monooleate | 0.5 |

(continued)

| Ingredient | | Amount (% by wt) |
|---|---|---|
| (9) | Polysiloxane-carrying amphiphilic urethane resin (G) (20% aqueous liquid) | 0.5 (0.1% as resin) |
| (10) | Oleyl alcohol | 0.5 |
| (11) | Placenta extract | 0.2 |
| (12) | Vitamin E acetate | 0.2 |
| (13) | Fragrance | Appropriate |
| (14) | Methylparaben | Appropriate |
| (15) | Trisodium edetate | Appropriate |
| (16) | Ion exchange water | Balance |

(Production method)

The components (4) and (5) were dissolved in a part of the component (16), in which then the components (1), (2) and (15) were dissolved successively. In the component (3), the components (7), (8), (10), (11), (12), (13) and (14) were dissolved successively, and then combined with the aqueous phase described above, and the mixture was microemulsified. Finally, the component (6) was dissolved in a part of the component (16) and added to the mixture, to which then the component (9) was added, and the mixture was stirred, deaerated and filtered. The resultant whitening essence was watery and smooth, and imparted elasticity to the skin.

[Example 20] Anti-UV essence

| Ingredient | | Amount (% by wt) |
|---|---|---|
| (1) | Stearic acid | 3.0 |
| (2) | Cetanol | 1.0 |
| (3) | Lanolin derivative | 3.0 |
| (4) | Liquid paraffin , | 5.0 |
| (5) | 2-Ethylhexyl stearate | 3.0 |
| (6) | 1,3-Butylene glycol | 6.0 |
| (7) | Polysiloxane-carrying amphiphilic urethane resin (H) (20% aqueous liquid) | 4.0 (0.8% as resin) |
| (8) | Polyoxyethylene cetyl alcohol ether | 2.0 |
| (9) | Glycerin monostearate | 2.0 |
| (10) | Triethanolamine | 1.0 |
| (11) | 2-Hydroxy-4-methoxybenzophenone | 4.0 |
| (12) | Dibenzoylmethane derivative | 4.0 |
| (13) | Sorbic acid | 0.2 |
| (14) | Fragrance | Appropriate |
| (15) | Ion exchange water | Balance |

(Production method)

The components (6), (7) and (10) were dissolved in the component (15) and kept at 70˚C by heating. The components (1) to (5) were dissolved by heating at 70 to 80˚C, and then the components (8), (9), (11), (12), (13) and (14) were added successively and the mixture was kept at 70˚C. The oil phase was added by stirring the aqueous phase described above to effect emulsification. The emulsion particle was made uniform using a homomixer, and then the mixture was deaerated, filtered and cooled. The resultant anti-UV essence was watery and smooth, and imparted suitable elasticity to the skin.

[Example 21] Hair styling gel

| Ingredient | | Amount (% by wt) |
|---|---|---|
| (1) | Carboxyvinyl polymer | 0.7 |
| (2) | Polysiloxane-carrying amphiphilic urethane resin (I) (20% aqueous liquid) | 5.0 (1.0% as resin) |
| (3) | Polysiloxane-carrying amphiphilic urethane resin (K) (20% aqueous liquid) | 5.0 (1.0% as resin) |
| (4) | Glycerin | 2.5 |
| (5) | 1,3-Butylene glycol | 2.5 |
| (6) | Polyoxyethylene hardened castor oil (40EO) | 0.5 |
| (7) | Dimethylpolysiloxane 100cs | 5.0 |
| (8) | Polyether-modified silicone (trade name: KF6011, produced by Shin-Etsu Chemical Co. Ltd.) | 1.0 |
| (9) | Sodium hydroxide | Appropriate (for adjusting at pH7.5) |
| (10) | Ethanol | 20.0 |
| (11) | Polyoxyethylene octyldodecyl ether | 0.1 |
| (12) | Fragrance | 0.1 |
| (13) | Trisodium edetate | 0.03 |
| (14) | Ion exchange water | Balance |

(Production method)

To the components (4), (5), (6), (8) and a part of (14), the component (7) was added and the mixture was emulsified. Then a part of the component (14) was added to form an emulsion part. On the other hand, the components (1), (2), (3), (9), (10), (11), (12) and (13) were dissolved uniformly in the remainder of the component (14), to which the emulsion part described above was added to obtain an emulsion type hair styling gel.

[Example 22] Nail polish

| Ingredient | | Amount (% by wt) |
|---|---|---|
| (1) | Ethyl carbitol | 2.5 |
| (2) | 1,3-Butylene glycol | 1.0 |
| (3) | Polysiloxane-carrying amphiphilic urethane resin (B) (20% aqueous liquid) | 45.0 (9.0% as resin) |
| (4) | Polysiloxane-carrying amphiphilic urethane resin (A) (20% aqueous liquid) | 40.0 (8.0% as resin) |
| (5) | Clay mineral | 0.2 |
| (6) | Polyoxyethylene hardened castor oil (40EO) | 0.3 |
| (7) | Colorant | Appropriate |
| (8) | Ion exchange water | Balance |

(Production method)

The component (6) was dissolved in the component (8), with which the component (7) was then mixed and dispersed thoroughly. Then the components (1) to (5) were mixed uniformly by stirring to obtain a nail polish.

[Example 23] Mascara

| | Ingredient | Amount (% by wt) |
|---|---|---|
| (1) | Iron oxide (black) | 10.0 |
| (2) | Polysiloxane-carrying amphiphilic urethane resin (C) (30% aqueous liquid) | 20.0 (6.0% as resin) |
| (3) | Polysiloxane-carrying amphiphilic urethane resin (G) (30% aqueous liquid) | 10.0 (3.0% as resin) |
| (4) | Solid paraffin | 8.0 |
| (5) | Lanolin wax | 8.0 |
| (6) | Light isoparaffin | 30.0 |
| (7) | Sesqui-oleic acid sorbitan | 4.0 |
| (8) | Ion exchange water | Balance |
| (9) | Preservative | Appropriate |
| (10) | Fragrance | Appropriate |

(Production method)

The component (8) was combined with the component (1), dispersed using a homomixer, admixed with the components (2) and (3), and kept at 70˚C by heating (aqueous phase). Other components were mixed and kept at 70˚C by heating (oil phase). The aqueous phase was dispersed uniformly in the oil phase using a homomixer.

[Example 24] Lip color

| | Ingredient | Amount (% by wt) |
|---|---|---|
| (1) | Titanium oxide | 4.5 |
| (2) | Red No.201 | 2.5 |
| (3) | Ceresin | 4.0 |
| (4) | Candelilla wax | 8.0 |
| (5) | Carnauba wax | 2.0 |
| (6) | Castor oil | 30.0 |
| (7) | Isostearic acid diglyceride | 40.0 |
| (8) | Polyoxyethylene hardened castor oil (20EO) | 1.0 |
| (9) | Ion exchange water | Balance |
| (10) | Polysiloxane-carrying amphiphilic urethane resin (D) (30% aqueous liquid) | 2.0 (0.6% as resin) |
| (11) | Polysiloxane-carrying amphiphilic urethane resin (E) (30% aqueous liquid) | 3.0 (0.9% as resin) |
| (12) | Glycerin | 2.0 |
| (13) | Fragrance | Appropriate |
| (14) | Antioxidant | Appropriate |

(Production method)

The components (1) and (2) were added to a part of the component (6), and treated using a roller (pigment part). The components (9) to (12) were mixed (aqueous phase). The components (3) to (5), a part of the component (6) and the components (7), (8) and (14) were mixed, melted by heating, combined with the pigment part at 80˚C, and mixed uniformly using a homomixer. Subsequently, water was added, and the mixture was emulsified and dispersed using a homomixer, combined with the component (13) and then poured into a mold.

INDUSTRIAL APPLICABILITY

According to the invention, an excellent cosmetic formulation capable of allowing the coating characteristics of a novel polysiloxane-carrying amphiphilic urethane resin to be exerted sufficiently can be obtained. For example, a hair cosmetic formulation in particular can give an improved hair elasticity without undergoing any flaking upon combing, and exhibits an excellent emulsion stability of the product. A makeup cosmetic formulation exhibits a watery touch and a long-lasting performance for example to its wearing resistance. A dermal cosmetic formulation is also watery and smooth and imparts suitable elasticity to the skin.

## Claims

**1.** A cosmetic formulation comprising an amphiphilic urethane resin tangling a polysiloxane compound obtainable by reacting at least compounds (A) to (D):

(A) a polyol compound;
(B) a polyisocyanate compound;
(C) a compound having at least one group selected from a hydroxyl group, primary amino group and secondary amino group and also having a carboxyl group; and
(D) a compound having at least one group selected from a hydroxyl group, primary amino group and secondary amino group and also having a tertiary amino group;
in the presence of a compound (S):
(S) a polysiloxane compound, said compound not being chemically bound with the amphiphilic urethane resin backbone by means of covalent bonds, wherein said compound is at least one selected from a dimethylpolysiloxane, polyether-modified silicone, cyclic silicone, phenyl-modified silicone, alkyl-modified silicone and alkoxy-modified silicone.

**2.** A cosmetic formulation according to Claim 1 wherein the amphiphilic urethane resin tangling a polysiloxane compound is an amphiphilic urethane resin obtainable by using at least the compounds (A) to (D) and (S)
by a method comprising a first step for producing an isocyanate group-containing prepolymer by reacting the compounds (A), (B) and (C) under an isocyanate group-excess condition and a second step for reacting said isocyanate group-containing prepolymer with the compound (D),
wherein the compound (S) is allowed to exist in at least one of the first step and second step.

**3.** A cosmetic formulation according to Claim 1 wherein the amphiphilic urethane resin tangling a polysiloxane compound is an amphiphilic urethane resin obtainable by using at least the compounds (A) to (D) and (S)
by a method comprising a first step for producing an isocyanate group-containing prepolymer by reacting the compounds (A), (B) and (D) under an isocyanate group-excess condition and a second step for reacting said isocyanate group-containing prepolymer with the compound (C),
wherein the compound (S) is allowed to exist in at least one of the first step and second step.

**4.** A cosmetic formulation according to Claim 1 wherein the amphiphilic urethane resin tangling a polysiloxane compound is obtainable by reacting in the presence of a compound (S) at least the compounds (A) to (E):
(E) a compound having at least one group selected from a hydroxyl group, primary amino group and secondary amino group and also having a structural unit represented by Formula (1):

$$-(C_2H_4O)_p(C_3H_6O)_q- \qquad (1)$$

wherein p is an integer of 1 to 500 and q is an integer of 0 to 400.

**5.** A cosmetic formulation according to Claim 4 wherein the amphiphilic urethane resin tangling a polysiloxane compound is obtainable by using at least the compounds (A) to (E) and (S)
by a method comprising a first step for producing an isocyanate group-containing prepolymer by reacting the compounds (A), (B), (C) and (E) under an isocyanate group-excess condition and a second step for reacting said isocyanate group-containing prepolymer with the compound (D),
wherein the compound (S) is allowed to exist in at least one of the first step and second step.

**6.** A cosmetic formulation according to Claim 4 wherein the amphiphilic urethane resin tangling a polysiloxane compound is an amphiphilic urethane resin obtainable by using at least the compounds (A) to (E) and (S) by a method comprising a first step for producing an isocyanate group-containing prepolymer by reacting the compounds (A), (B), (D) and (E) under an isocyanate group-excess condition and a second step for reacting said isocyanate group-containing prepolymer with the compound (C), wherein the compound (S) is allowed to exist in at least one of the first step and second step.

**7.** A cosmetic formulation according to any one of Claims 1 to 6 wherein the cosmetic formulation is a hair cosmetic formulation.

**8.** A cosmetic formulation according to any one of Claims 1 to 6 wherein the cosmetic formulation is a dermal external formulation.

**Patentansprüche**

**1.** Kosmetische Zubereitung, umfassend ein amphiphiles Urethanharz, das mit einer Polysiloxanverbindung verknäuelt ist, erhältlich durch Umsetzen von mindestens der Verbindungen (A) bis (D):

(A) eine Polyolverbindung;
(B) eine Polyisocyanatverbindung;
(C) eine Verbindung mit mindestens einer Gruppe, gewählt aus einer Hydroxylgruppe, primären Aminogruppe und sekundären Aminogruppe und welche ebenso eine Carboxylgruppe besitzt; und
(D) eine Verbindung mit mindestens einer Gruppe, gewählt aus einer Hydroxylgruppe, primären Aminogruppe und sekundären Aminogruppe, und welche ebenso eine tertiäre Aminogruppe besitzt;
in Gegenwart einer Verbindung (S):
(S) eine Polysiloxanverbindung, wobei diese Verbindung nicht mit dem Grundgerüst des amphiphilen Urethanharzes mittels kovalenter Bindungen chemisch verbunden ist, wobei die Verbindung mindestens eine ist, gewählt aus einem Dimethylpolysiloxan, Polyether-modifizierten Silicon, cyclischen Silicon, Phenyl-modifizierten Silicon, Alkylmodifizierten Silicon und Alkoxy-modifizierten Silicon.

**2.** Kosmetische Zubereitung nach Anspruch 1, wobei das amphiphile Urethanharz, das mit einer Polysiloxanverbindung verknäuelt ist, ein amphiphiles Urethanharz ist, erhältlich durch Verwendung von mindestens der Verbindungen (A) bis (D) und (S)
durch ein Verfahren, umfassend einen ersten Schritt zur Erzeugung eines Isocyanatgruppen enthaltenden Prepolymeren durch Umsetzen der Verbindungen (A), (B) und (C) unter der Bedingung eines Isocyanatgruppenüberschusses, und einen zweiten Schritt zur Umsetzung des Isocyanatgruppen enthaltenden Prepolymeren mit der Verbindung (D),
wobei die Verbindung (S) bei mindestens einem aus dem ersten Schritt und dem zweiten Schritt vorliegt.

**3.** Kosmetische Zubereitung nach Anspruch 1, wobei das amphiphile Urethanharz, das mit einer Polysiloxanverbindung verknäuelt ist, ein amphiphiles Urethanharz ist, erhältlich durch Verwendung von mindestens der Verbindungen (A) bis (D) und (S)
durch ein Verfahren, umfassend einen ersten Schritt zur Erzeugung eines Isocyanatgruppen enthaltenden Prepolymeren durch Umsetzen der Verbindungen (A), (B) und (D) unter der Bedingung eines Isocyanatgruppenüberschusses, und einen zweiten Schritt zur Umsetzung des Isocyanatgruppen enthaltenden Prepolymeren mit der Verbindung (C),
wobei die Verbindung (S) bei mindestens einem aus dem ersten Schritt und dem zweiten Schritt vorhanden ist.

**4.** Kosmetische Zubereitung nach Anspruch 1, wobei das amphiphile Urethanharz, das mit einer Polysiloxanverbindung verknäuelt ist, erhältlich ist durch Umsetzen, in Gegenwart einer Verbindung (S), von mindestens der Verbindungen (A) bis (E):

(E) eine Verbindung mit mindestens einer Gruppe, gewählt aus einer Hydroxylgruppe, primären Aminogruppe und sekundären Aminogruppe, und welche ebenso eine Struktureinheit der Formel (1) besitzt:

$$-(C_2H_4O)_p(C_3H_6O)_q- \qquad (1)$$

worin p eine ganze Zahl von 1 bis 500 und q eine ganze Zahl von 0 bis 400 ist.

5. Kosmetische Zubereitung nach Anspruch 4, wobei das amphiphile Urethanharz, das mit einer Polysiloxanverbindung verknäuelt ist, erhältlich ist durch Verwendung von mindestens der Verbindungen (A) bis (E) und (S)
durch ein Verfahren, umfassend einen ersten Schritt zur Erzeugung eines Isocyanatgruppen enthaltenden Prepolymeren durch Umsetzen der Verbindungen (A), (B), (C) und (E) unter der Bedingung eines Isocyanatgruppenüberschusses, und einen zweiten Schritt zur Umsetzung des Isocyanatgruppen enthaltenden Prepolymeren mit der Verbindung (D),
wobei die Verbindung (S) während mindestens einem aus dem ersten Schritt und dem zweiten Schritt vorhanden ist.

6. Kosmetische Zubereitung nach Anspruch 4, wobei das amphiphile Urethanharz, das mit einer Polysiloxanverbindung verknäuelt ist, ein amphiphiles Urethanharz ist, erhältlich durch Verwendung von mindestens der Verbindungen (A) bis (E) und (S)
durch ein Verfahren, umfassend einen ersten Schritt zur Erzeugung eines Isocyanatgruppen enthaltenden Prepolymeren durch Umsetzen der Verbindungen (A), (B), (D) und (E) unter der Bedingung eines Isocyanatgruppenüberschusses, und einen zweiten Schritt zur Umsetzung des Isocyanatgruppen enthaltenden Prepolymeren mit der Verbindung (C),
wobei die Verbindung (S) während mindestens einem aus dem ersten Schritt und dem zweiten Schritt vorhanden ist.

7. Kosmetische Zubereitung nach mindestens einem der Ansprüche 1 bis 6, wobei die kosmetische Zubereitung eine kosmetische Haarzubereitung ist.

8. Kosmetische Zubereitung nach mindestens einem der Ansprüche 1 bis 6, wobei die kosmetische Zubereitung eine externe Dermalzubereitung ist.

**Revendications**

1. Une composition cosmétique comprenant une résine uréthane amphiphile enchevêtrée avec un composé polysiloxane pouvant être obtenue en faisant réagir au moins les composés (A) à (D) :

   (A) un composé polyol ;
   (B) un composé polyisocyanate ;
   (C) un composé ayant au moins un groupe choisi parmi un groupe hydroxyle, un groupe amino primaire, un groupe amino secondaire et ayant également un groupe carboxylique ; et
   (D) un composé ayant au moins un groupe choisi parmi un groupe hydroxyle, un groupe amino primaire, un groupe amino secondaire et ayant également un groupe amino tertiaire ; en présence d'un composé (S) :
   (S) un composé polysiloxane, ledit composé n'étant pas lié chimiquement avec la résine uréthane amphiphile constituant l'épine dorsale au moyen de liaisons covalentes, ledit composé étant au moins l'un des composés choisis parmi le diméthylpolysiloxane, la silicone modifiée par polyéther, la silicone cyclique, la silicone modifiée par phényle, la silicone modifiée par alkyle et la silicone modifiée par alkoxy.

2. Une composition cosmétique selon la revendication 1, dans laquelle la résine uréthane amphiphile enchevêtrée avec un composé polysiloxane est une résine uréthane amphiphile pouvant être obtenue en utilisant au moins les composés (A) à (D) et (S), par un procédé comprenant une première étape pour produire un prépolymère contenant un groupe isocyanate en faisant réagir les composés (A), (B) et (C) dans une condition d'excès du groupe isocyanate et une seconde étape pour faire réagir ledit prépolymère contenant un groupe isocyanate avec le composé (D), le composé (S) pouvant exister au moins dans l'une des première et seconde étapes.

3. Une composition cosmétique selon la revendication 1 dans laquelle la résine uréthane amphiphile enchevêtrée avec un composé polysiloxane est une résine uréthane amphiphile pouvant être obtenue en utilisant au moins les composés (A) à (D) et (S), par un procédé comprenant une première étape pour produire un prépolymère contenant un groupe isocyanate en faisant réagir les composés (A), (B) et (D) dans une condition d'excès de groupe isocyanate et une seconde étape pour faire réagir ledit prépolymère contenant un groupe isocyanate avec le composé (C), le composé (S) pouvant exister au moins dans l'une des première et seconde étapes.

4. Une composition cosmétique selon la revendication 1 dans laquelle la résine uréthane amphiphile enchevêtrée avec un composé polysiloxane est une résine uréthane amphiphile pouvant être obtenue en utilisant au moins les

composés (A) à (E) :

(E) un composé ayant au moins un groupe choisi parmi un groupe hydroxyl, un groupe amine primaire et un groupe amine secondaire et ayant également une unité structurelle représentée par la formule (1)

$$-[C_2H_4O]_p(C_3H_6O)_q- \qquad (1)$$

dans laquelle p est un nombre entier compris entre 1 et 500 et q est un nombre entier compris entre 0 et 400.

**5.** Une composition cométique selon la revendication 4, dans laquelle la résine uréthane amphiphile enchevêtrée avec un composé polyoxilane est une résine uréthane amphiphile pouvant être obtenue en utilisant au moins les composés (A) à (E) et (S), par un procédé comprenant une première étape pour produire un prépolymère contenant un groupe isocyanate en faisant réagir les composés (A), (B) (C) et (E) dans une condition d'excès de groupe isocyanate et une seconde étape pour faire réagir ledit prépolymère contenant un groupe isocyanate avec le composé (D), le composé (S) pouvant exister au moins dans l'une des première et seconde étapes.

**6.** Une composition cosmétique selon la revendication 1 dans laquelle la résine uréthane amphiphile enchevêtrée avec un composé polysiloxane est une résine uréthane amphiphile pouvant être obtenue en utilisant au moins les composés (A) à (E) et (S), par un procédé comprenant une première étape pour produire un prépolymère contenant un groupe isocyanate en faisant réagir les composés (A), (B), (D) et (E) dans une condition d'excès du groupe isocyanate et un seconde étape pour faire réagir ledit prépolymère contenant avec le composé (C), le composé (S) pouvant exister au moins dans l'une des premières et seconde étapes.

**7.** Une composition cosmétique selon l'une des revendications 1 à 6, dans laquelle la composition cosmétique est une composition cosmétique pour les cheveux.

**8.** Une composition cosmétique selon l'une des revendications 1 à 6, dans laquelle la composition cométique est une composition dermique extérieure.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11228363 A
- JP 2000191476 A
- JP 2001048735 A